# EUROPEAN PATENT SPECIFICATION

(11) **EP 2 978 861 B1**
(45) Date of publication and mention of the grant of the patent: **15.07.2020**
(21) Application number: 14729760.0
(22) Date of filing: 27.03.2014
(51) Int. Cl.: C12Q 1/6886

(54) **UNBIASED DNA METHYLATION MARKERS DEFINE AN EXTENSIVE FIELD DEFECT IN HISTOLOGICALLY NORMAL PROSTATE TISSUES ASSOCIATED WITH PROSTATE CANCER: NEW BIOMARKERS FOR MEN WITH PROSTATE CANCER**
UNBEEINFLUSSTE DNA-METHYLIERUNGSMARKER ZUR DEFINITION EINES FELDDEFEKTES BEI HISTOLOGISCH NORMALEM PROSTATAGEWEBE IM ZUSAMMENHANG MIT PROSTATAKREBS: NEUE BIOMARKER FÜR MÄNNER MIT PROSTATAKREBS
DES MARQUEURS DE MÉTHYLATION DE L'ADN SANS BIAIS DÉFINISSENT UN DÉFAUT DE CHAMP EXTENSIF DANS DES TISSUS DE LA PROSTATE HISTOLOGIQUEMENT NORMAUX ASSOCIÉS AU CANCER DE LA PROSTATE : NOUVEAUX BIOMARQUEURS POUR DES HOMMES ATTEINTS DU CANCER DE LA PROSTATE

(30) Priority: 28.03.2013 US 201361806218 P; 29.03.2013 US 201361806566 P
(43) Date of publication of application: 03.02.2016
(73) Proprietor: Wisconsin Alumni Research Foundation, Madison, WI 53705 (US)
(72) Inventor: JARRARD, David, Frazier, Madison, WI 53705 (US); YANG, Bing, Madison, WI 53717 (US)
(74) Representative: J A Kemp LLP
(86) International application number: PCT/US2014/031957
(87) International publication number: WO 2014/160829

(56) References cited:
- US-A1- 2012 135 877
- MATTHEW TRUONG ET AL: "Using the Epigenetic Field Defect to Detect Prostate Cancer in Biopsy Negative Patients", THE JOURNAL OF UROLOGY, vol. 189, no. 6, 15 November 2012 (2012-11-15), pages 2335-2341, XP055141138, ISSN: 0022-5347, DOI: 10.1016/j.juro.2012.11.074 cited in the application & Matthew Truong ET AL: "Using the Epigenetic Field Defect to Detect Prostate Cancer in Biopsy Negative Patients", THE JOURNAL OF UROLOGY, 15 November 2012 (2012-11-15), pages 2335-2341, XP055141145, DOI: 10.1016/j.juro.2012.11.074 Retrieved from the Internet: URL:http://www.sciencedirect.com/science/a rticle/pii/S0022534712055681 [retrieved on 2014-09-18]

## Description

### CROSS-REFERENCE TO RELATED APPLICATION

[Deleted]

[Deleted]

### BACKGROUND

It is estimated that 198,280 men were diagnosed with prostate cancer and 27,360 men died from prostate cancer (PCa) in 2009 in the USA (Jemal et al., (2009) CA Cancer J Clin 59, 225-249). The predominant tools for early detection of prostate cancer are prostate specific antigen (PSA) testing and digital rectal exam (DRE). However, 65% to 70% of men with total PSA ranging between 4.0-10.0 ng/ml have a negative prostate biopsy result. In addition, 15% of PCa patients have PSA levels < 4.0 ng/ml, indicating a weak predictive ability (Thompson et al., (2004) N Engl J Med 350, 2239-2246). PSA-based screening also detects non-significant cancers leading to an estimated 50% of overdiagnosis (Fritz et al., (2009) The New England Journal of Medicine 360). A urine-based test examining an RNA molecule termed PCA-3 is currently undergoing FDA trials. Prostate biopsy is used to confirm disease. However, because of sampling errors repeated sets of samples are commonly required to make a diagnosis (Gann et al., (2010) JCO 28, 7). Typical biopsy schemes include 10-12 or more tissue cores removed under local anesthetic. Re-biopsy is often required two to three times in order to rule out cancer because of sampling errors. Cancers can also be missed because of sampling problems.

There is a clear need for biomarkers that allow easier and more accurate diagnosis and prognosis of prostate cancer. Also referred to are:
- US2012/135877 A1 which is concerned with DNA markers for prostate cancer field defect; and
- Truong et al (2012) J. Urol. 189(6):2335-2341 which concerned with using epigenetic field defect to detect prostate cancer in biopsy negative patients.

### SUMMARY OF THE INVENTION

In one embodiment, the present invention provide a method of diagnosing prostate cancer in a human subject comprising quantitating methylation in genomic DNA obtained from the human subject in at least one target region selected from the group consisting of SEQ ID NO: 18 and SEQ ID NO:39, wherein significant methylation changes indicate the presence of prostate cancer or a prostate cancer field defect, wherein the change is relative to tissue from a second human subject who does not have prostate cancer. Also provided is such a method where at least two, three or four regions are selected from the group consisting of CAV1, EVX1, MCF2L, FGF1, NCR2, WNT2, EXT1 and SPAG4 target regions wherein at least EXT1 or SPAG4 are selected. Preferably, the significant methylation change is p<0.05 or at least ±50% of the pyrosequencing percentages or fold-changes shown in Table 1.

Also disclosed is the amplification product described above.

Also disclosed is a combination of the amplification product described above and materials useful to determine methylation status.

In another embodiment, the genomic DNA is obtained from prostate tissue. In another embodiment, the genomic DNA is obtained from body fluid preferably selected from the group consisting of urine and semen. Most preferably the bodily fluid is urine.

In a preferred embodiment, primer sets are used for amplification of the target region and at least one primer within each set of primers is biotinylated.

In yet another preferred embodiment, the methylation is quantified via pyrosequencing.

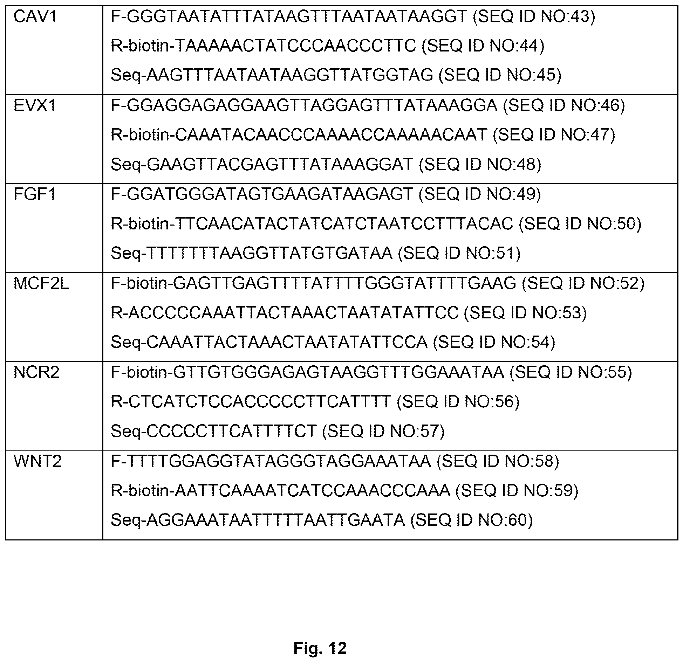

Also disclosed is the quantitation of methylation which comprises analyzing whether the CAV1, EVX1 or MCF2L regions are hypermethylated or FGF1, WNT2 or NCR2 regions are hypomethylated as a positive correlation to prostate cancer field defect. Preferably, the target loci comprise sequences selected from the group consisting of SEQ ID Nos:1-6. Preferably, the target loci are amplified using at least one set of primers in Fig. 12.

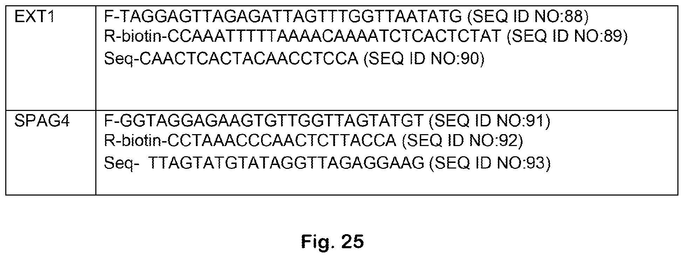

In another embodiment, the quantitation of methylation comprises analyzing whether the target region of SEQ ID NO: 39 is hypermethylated and the target region of SEQ ID NO: 18 is hypomethylated as a positive correlation to prostate cancer field defect. Preferably, the target loci are amplified using at least one set of primers in Fig. 25.

In another embodiment, the quantitation of methylation comprises analyzing whether the target region of SEQ ID NO: 39 is hypermethylated or the target region of SEQ ID NO: 18 is hypomethylated as a positive correlation to prostate cancer field defect. Whether CAV1, EVX1 or MCF2L is hypermethylated or FGF1, WNT2 or NCR2 is hypomethylated may additionally be analysed those target loci having the sequences of SEQ ID Nos:1-6, 18 and 39. Preferably, the target loci are amplified using at least one set of primers in Figs. 12 and 25.

In another embodiment, the human subject is a prostate cancer patient.

Also disclosed is a method of diagnosing high grade prostate cancer field defect in a human subject comprising the steps of: (a) quantitating the methylation in genomic DNA obtained from a human subject in at least one target region selected from the group consisting of NCR2 and WNT2 target, wherein significant methylation changes indicate the presence of high grade prostate cancer field defect or prostate cancer, wherein the change is relative to tissue from a second human subject who does not have prostate cancer; and (b) treating the human subject for high grade prostate cancer field defect based the results of steps (a) and (b).

Also disclosed is a method of screening biomarkers for prostate cancer comprising the steps of: (a) quantitating the methylation in genomic DNA obtained from a human subject in at least one target region selected from the group consisting of SEQ ID NOs:1-6 and 18, 39; wherein significant methylation changes indicate the presence of prostate cancer field defect or prostate cancer, wherein the change is relative to tissue from a second human subject who does not have prostate cancer.

Also disclosed is a method of screening biomarkers for prostate cancer comprising the steps of: (a) quantitating the methylation in genomic DNA obtained from a human subject in at least one target region selected from the group consisting of SEQ ID NOs:61-77 and 94-97; wherein significant methylation changes indicate the presence of prostate cancer field defect or prostate cancer, wherein the change is relative to tissue from a second human subject who does not have prostate cancer.

Other objects, advantages and features of the present invention will become apparent from the following specification taken in conjunction with the accompanying drawings.

### BRIEF DESCRIPTION OF DRAWINGS

This patent application file contains at least one drawing executed in color. Copies of this patent or patent application publication with color drawings will be provided by the Office upon request and payment of the necessary fee.

The invention will be better understood and features, aspects and advantages other than those set forth above will become apparent when consideration is given to the following detailed description thereof. Such detailed description makes reference to the following drawings, wherein:
Fig. 1 shows the sequence of the target region for CAV1 (SEQ ID NO:1).
Fig. 2 shows the sequence of the target region for EVX1 (SEQ ID NO:2).
Fig. 3 shows the sequence of the target region for MCF2L (SEQ ID NO:3).
Fig. 4 shows the sequence of the target region for FGF1 (SEQ ID NO:4).
Fig. 5 shows the sequence of the target region for NCR2 (SEQ ID NO:5).
Fig. 6 shows the sequence of the target region for WNT2 (SEQ ID NO:6).
Fig. 7 shows probe sequences used in the methylation array for the genes CAV1, EVX1, MCF2L, FGF1, NCR2 and WNT2.
Fig. 8 is a diagram demonstrating microdissection of prostate tissue.

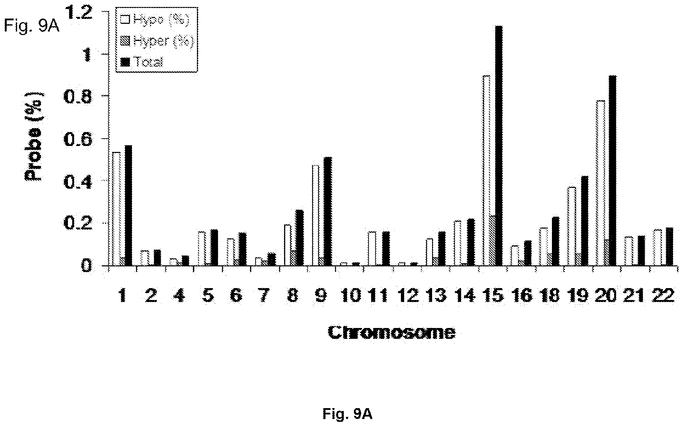

Fig. 9A shows genome-wide distribution of DNA methylation array differences at 385,000 loci in histologically normal tumor-associated (TA) prostate tissues compared to non-tumor associated (NTA) tissues. Significant differences in methylation between TA and NTA prostate tissues were generated using a cut-off of probe score of -log10 [p] that ranged from 2 to 10 resulting in around 1,000 probes on each chromosome and 18,101 probes in total. After statistical analysis comparing the log₂-ratios between the NTA and TA groups, significant methylation differences between groups were determined using a t-test (P < 0.05). A total of 615 probes were differentially methylated in TA tissues with 537 demonstrating hypomethylation and 78 hypermethylation. The percentage (axis) is the significantly altered probe number versus the total probe number analyzed for each chromosome. Chromosomes 15 and 20 were differentially methylated to a greater extent than other chromosomes.
Fig. 9B shows the significant methylation changes across 41,522,036-4,2004,151 on chromosome 15p. The data are represented as a ratio of Mean TA/ NTA.
Fig. 9C shows the significant methylation changes across 33,343,402-33,565,080 on chromosome 20p. The data are represented as ratio of Mean TA/ NTA.
Fig. 9D is a heat map of significant DNA methylation array changes using unsupervised hierarchical clustering. Using more stringent criteria (t-test, p < 0.01), 87 probes are shown comparing sets of NTA (left) to TA (right) and hierarchically ordered from top to bottom by relatively hypermethylation to hypomethylation. Green indicates relative hypomethylation whereas the red shaded areas demonstrate hypermethylation. The heat map was generated with JAVA TMEV™ (MultiExperiment View).
Fig. 10 is a schematic representation of CpGs analyzed by Pyrosequencing. The ratio of ObsCpG/ExpCpG and GC percentage for all regions are: CAV1 1.2, 60%; EVX1 0.8, 60%; FGF1 1.0, 50%; MCF2L 1.0, 60%; NCR2 0.5, 50%; WNT2 1.0, 50%.

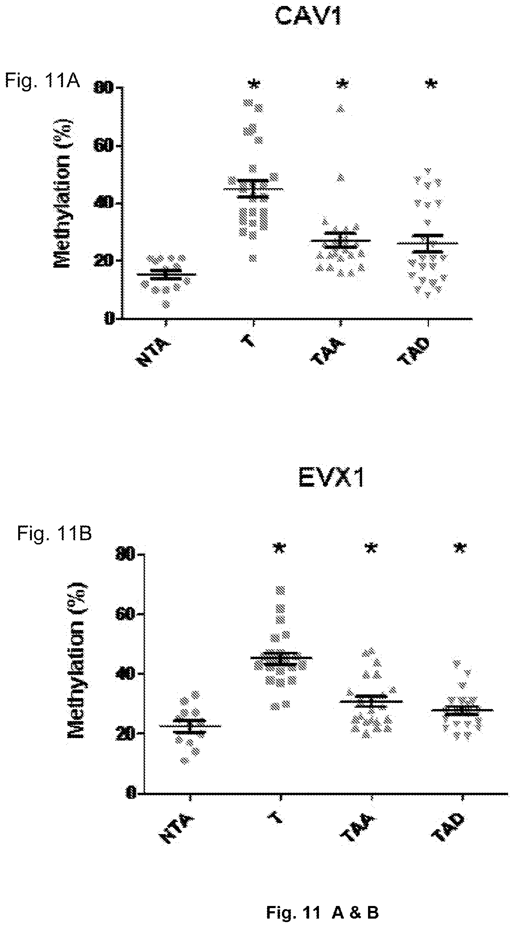

Fig. 11A-D shows CAV1, EVX1, MCF2L and FGF1 methylations. To analyze CAV1 methylation, we analyzed methylation of ten CpGs and eight out of the ten CpGs showed significantly increased methylation in T (tumor), TAA (tumor-associated adjacent) and TAD (tumor-associated distant) prostate tissue compared to NTA (non-tumor-associated normal prostate tissue). The figure shows methylation percentages of the sixth CpG and they are 14%, 45%, 27% and 26% for NTA, T, TAA and TAD prostate tissues, respectively. *t-test. P < 0.05 was used for all figures below. To analyze EVX1 methylation, we tested six CpGs for EVX1 and four out of the six showed significantly increased methylation in T, TAA and TAD compared to NTA prostate tissues. This figure shows methylation percentage of the third CpG and they are 22%, 45%, 31% and 28% for NTA, T, TAA and TAD prostate tissues, respectively. For MCF2L, the region detected contains nine CpGs and three out of the nine CpGs showed significantly increased methylation in T, TAA and TAD compared to NTA prostate tissue. This figure shows the methylation for the first CpG and they are 80%, 88%, 85% and 85% for NTA, T, TAA and TAD prostate tissues, respectively. For FGF1, all four CpGs we analyzed showed significantly decreased methylation in TAA and TAD compared to NTA prostate tissue, but no significant change in T prostate tissue. This figure shows methylation percentage of the third CpG and they are 71%, 73%, 60% and 61% for NTA, T, TAA and TAD prostate tissues, respectively.

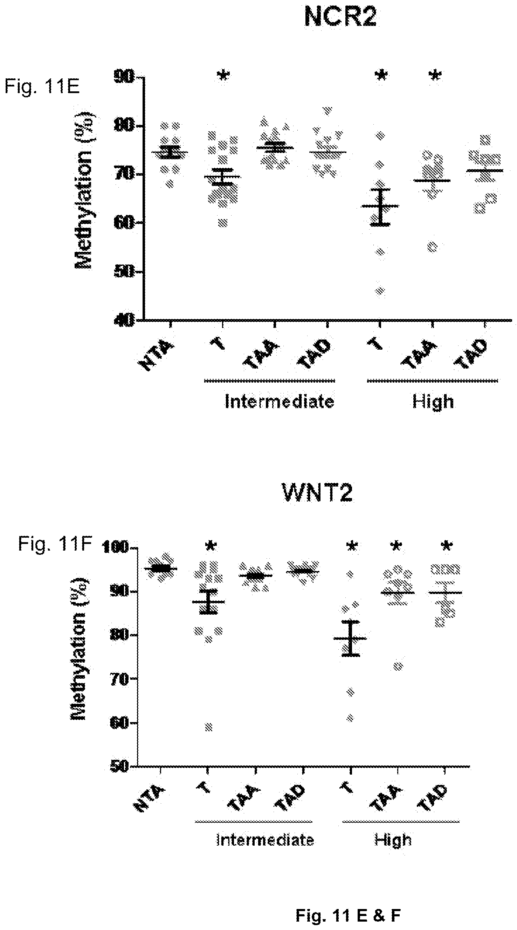

Fig. 11E-F shows NCR2 and WNT2 methylations. For NCR2, three CpGs were analyzed within the target region. In the prostate with high grade (Gleason grade ≥ 8, H) the third CpG showed significantly decreased methylation in T and TAA prostate compared to NTA prostate tissue. However, in the prostate with intermediate grade (Gleason grade 6 & 7, Int), the methylation change of this CpG was only significant in T prostate. This figure shows methylation of the third CpG and they are 75%, 69%, 63%, 68% and 70% for NTA, T (Int), T (H), TAA(H) and TAD(H), respectively. For WNT2, we detected methylation of four CpGs. In the prostate with high grade, two of them showed significantly decreased methylation in all T, TAA and TAD prostate tissues compared to NTA prostate tissue. However, in the prostate with intermediate grade, methylation change was only significant in T prostate tissue. This figure shows methylation of the first CpG and they are 95%, 87%, 79%, 89% and 89% for NTA, T (Int), T (H), TAA (H) and TAD (H), respectively.
Fig. 12 shows the sequences of primers used for pyrosequencing.
Fig. 13 shows AMACR expression in NTA, T, TAA and TAD prostate tissues which will be used in quantitative methylation Pyrosequencing. AMACR expression was assayed with quantitative RT-PCR, the data are shown as ΔCT. Two NTA and three TA (T,TAA,TAD) specimens were excluded from experiential group due to higher AMACR expression.
Fig. 14 shows the sequence of the expanded region of CAV1 to screen for methylation changes associated with PCa.
Fig. 15 shows the sequence of the expanded region of EVX1 to screen for methylation changes associated with PCa.
Fig. 16 shows the sequence of the expanded region of MCF2L to screen for methylation changes associated with PCa.
Fig. 17 shows the sequence of the expanded region of FGF1 to screen for methylation changes associated with PCa. Since there is no CPG island within the promoter region, all the regions shown are within introns between exons one and three.
Fig. 18 shows the sequence of the expanded region of NCR2 to screen for methylation changes associated with PCa.
Fig. 19 shows the sequence of the expanded region of WNT2 to screen for methylation changes associated with PCa.
Fig. 20 shows the sequence of the target region for EXT1 (SEQ ID NO:18).
Fig. 21 shows the sequence of the target region for SPAG4 (SEQ ID NO:39).
Fig. 22 shows probe sequences used in the methylation array for the genes EXT1 and SPAG4 (SEQ ID NOs:86-87).
Fig. 23 is a schematic representation of CpGs analyzed by Pyrosequencing. The ratio of ObsCpG/ExpCpG and GC percentage for all regions are: EXT1 0.8, 60%; SPAG4 0.55, 60%.

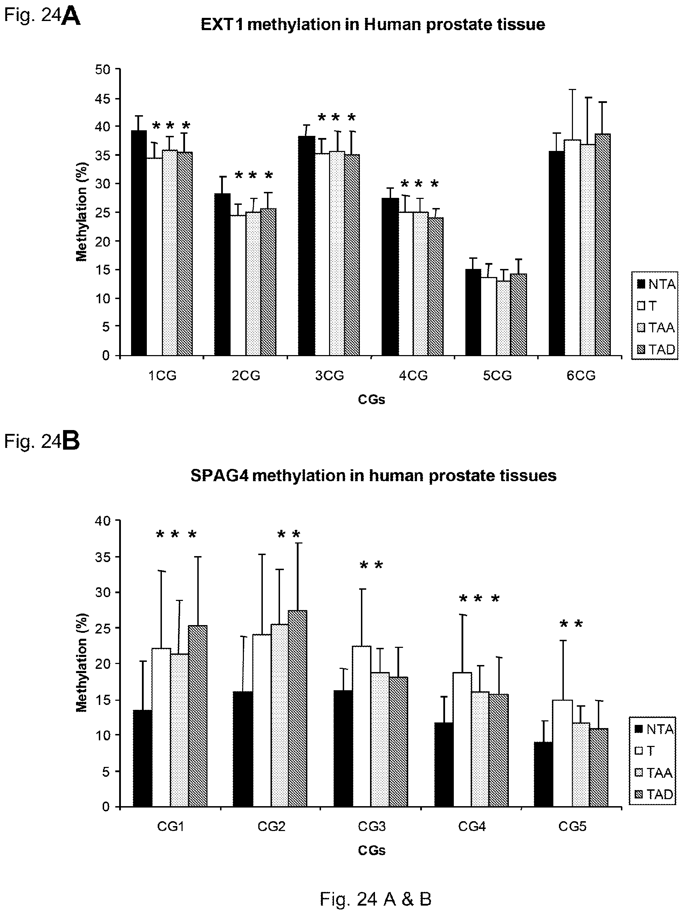

Fig. 24 shows EXT1 and SPAG4 methylations. To analyze EXT1 methylation, we analyzed methylation of six CpGs and four out of the six CpGs showed significantly increased methylation in T (tumor), TAA (tumor-associated adjacent) and TAD (tumor-associated distant) prostate tissue compared to NTA (non-tumor-associated normal prostate tissue). The figure shows methylation percentages of all six CpGs. *t-test. P < 0.05 was used for all figures below. To analyze SPAG4 methylation, we tested five CpGs for SPAG4 and five out of the five showed significantly increased methylation in T, TAA and TAD compared to NTA prostate tissues. This figure shows methylation percentage of the all five CpGs.
Fig. 25 shows the sequences of primers used for target amplification and pyrosequencing (SEQ ID NOs:88-93).
Fig. 26 shows the sequence of the expanded region of EXT1 to screen for methylation changes associated with PCa (SEQ ID NO:94).
Fig. 27 shows the sequence of the expanded region of SPAG4 to screen for methylation changes associated with PCa (SEQ ID NOs:95-97).
Fig. 28 shows methylation of the EVX1, CAV1, FGF1 and NCR2 in urine from the patients with positive or negative biopsies for prostate cancer.
Fig. 29 shows DNA isolation from paraffin-embedded prostate biopsies.

### DESCRIPTION OF THE PRESENT INVENTION

### In General

Like other human cancers, prostate cancer development and progression is driven by the interplay of genetic and epigenetic changes (Schulz et al., (2009) Semin Cancer Biol 19, 172-180). Changes in somatic DNA methylation constitute a superb source of cancer biomarkers for several reasons. These changes can be detected using PCR methods at single-copy sensitivity and small DNA fragments are more stable in blood and body fluids than RNA or protein species. In addition, acquired DNA methylation differences have been reported for nearly every human cancer. Finally, somatic hypermethylation of CpG island sequences may be more consistent for a given cancer than genetic changes (Nelson et al., (2009) Endocrinology 150, 3991-4002). Patterns of DNA methylation in tumors may also discriminate aggressive vs. nonaggressive disease and predict responsiveness to specific treatments (Nelson et al., (2009) Endocrinology 150, 3991-4002).

Genetic and epigenetic alterations do not appear to be limited to the cancerous cells, as recent data indicates tissue adjacent or distant to the tumor is also abnormal (Nonn et al., (2009) Prostate 69, 1470-1479). This field defect (also termed field effect) has been identified in colon and head and neck cancer, as well as prostate based on alterations in gene expression (YP, Y. (2004) Journal of Clinical Oncology 22; Chandran et al., (2005) BMC Cancer 5, 45) and genomic loss of imprinting (Agnieszka et al., (2009) International Journal Of Oncology 35, 87-96). Aberrant methylation patterns in the GSTP1, RARb2, APC and RASSF1A promoters have been detected in normal epithelial or stromal tissue adjacent to cancer (Aitchison et al., (2007) Prostate 67, 638-644; Hanson et al., (2006) J. Natl. Cancer Inst. 98, 255-261; Henrique et al., (2006) Mol Cancer Res 4, 1-8). These genes are altered in the tumor and represent a single gene approach to analyzing the field effect. Results vary as to whether this field effect is limited to the tissue adjacent to the tumor or whether it is found in distant 'normal' tissue.

By use of the present invention, one can reassure men who have a negative biopsy that no cancer is present by testing for the presence of the field defect without additional future biopsies and avoid the complications directly associated with increasing the biopsy number and frequency. If methylation changes associated with a biopsy field defect are detected, more detailed imaging with an MRI and endorectal probe and a more aggressive detection strategy requiring anesthesia and 30-50 biopsies will typically be undertaken to detect and/or characterize the disease. This approach is associated with additional risks associated with anesthesia, infection, bleeding and others, and is not performed routinely. In addition, it is likely these patients would be monitored much more closely.

In developing the present invention, the inventors have analyzed histologically normal tissues from men with and without prostate cancer utilizing a high-throughput technique that simultaneously scans 385,000 regions of the genome. Using a human ENCODE methylation array (Roche Nimblegen), the inventors have found distinct alterations in methylation at specific loci or "target regions". The inventors associated methylation changes at these loci with the presence of prostate cancer. Analysis of these loci in tissue samples from patients will enhance the detection of prostate cancer.

By "histologically normal", we mean prostate tissue that has no evidence of disease in the specimen itself, based on standard morphologic and histochemical criteria used by pathology. By "normal" or "non-tumor associated (NTA)", we mean prostate specimen which not only does not contain cancer itself, as defined by a pathologist, but also does not contain cancer elsewhere in the prostate. By "tumor associated (TA)", we mean a prostate specimen which does not show evidence of cancer, but is taken from a prostate with evidence of cancer in another location. One would appreciate that both "non-tumor associated" and "tumor associated" prostate specimens in this application are "histologically normal" prostate specimens.

Standard PCR methods generally entail amplification of a target region using a pair of forward and reverse primers that are designed to be complementary to sequences flanking the target region. The size of a fragment that can be amplified using PCR can range from less than 50 base pairs (bp) to greater than 10,000 base pairs. Similarly, sequencing of a target region can be accomplished by designing sequencing primers that are complimentary to a sequence less than 50 bp upstream of the target gene or more than 1000 bp upstream depending on the sequencing technology selected. Therefore it is possible to design many permutations of sequencing primers or PCR primer sets that are capable of amplifying a given target region. For example, given a sample containing genomic DNA comprising a 500 bp target gene or region, a primer set can be designed to amplify i) the explicit target region; or ii) a region encompassing the target region including upstream and downstream sequence. If the minimum requirement is a 20 bp primer and the amplified fragment size can range from 500 to 10,000bp, the number of potential primer sets that can be used to amplify the target region is on the order of 10⁴.

A number of preferred primers for amplification of specific target regions are disclosed and may be employed. However, one skilled in the art will appreciate that the target regions disclosed in the present invention can be amplified by other than the described primers, which have been presented for purposes of illustration. A number of PCR amplification and sequencing schemes are contemplated.

### Biomarker Candidates

The inventors identified eight biomarker candidates associated with the genes CAV1, EVX1, MCF2L, FGF1, WNT2, NCR2, EXT1 and SPAG4 which showed significant changes (p<0.05) in methylation in target regions when normal and tumor-associated tissues are compared (Table 1). The CAV1, EVX1, MCF2L and SPAG4 regions showed hypermethylation, and the FGF1, WNT2, NCR2 and EXT1 regions showed hypomethylation.

**Table 1**

| **Gene** | **Location** | **Function** | **Fold Change Microarray** | **Pyrosequencing** |
|---|---|---|---|---|
| CAV1 | 7q31.1 | Tumor suppressor gene candidate | 7.6 | 30% increased in tumor, 12% in tumor-associated, adjacent and distant |
| | | A negative regulator of the Ras-p42/44 MAP kinase cascade | | |
| | | Negative regulation of JAK-STAT cascade | | |
| | | A scaffolding protein within caveolar membranes | | |
| EVX1 | 7p15-p14 | Sequence-specific DNA binding, transcription factor | 7.1 | 23% increased in tumor, 6-13% in tumor-associate, adjacent and distant |
| | | A role in the specification of neuronal cell types. | | |
| FGF1 | 5q31 | Fibroblast growth factor receptor signaling pathway | 0.77 | 11-15% decreased in tumor-associated, adjacent and distant |
| | | Positive regulation of epithelial cell proliferation | | |
| | | Embryonic development, cell growth, tumor growth and invasion | | |
| MCF2L | 13q34 | Rho guanine nucleotide exchange factor activity | 4.5 | 8% increased in tumor, 5% in tumor-associated, adjacent and distant |
| NCR2 | 6p21.1 | Increases efficiency of activated NK cells | 0.6 | 11% decreased in tumor, adjacent and distant for high grade |
| | | To mediate tumor cell lysis | | 5% decreased in tumor for intermediate grade |
| WNT2 | 7q31.2 | Wnt receptor signaling pathway, calcium modulating pathway | 0.7 | 16% decreased in tumor, 5% in adjacent and distant for high grade |
| | | Implicated in oncogenesis and in several developmental processes (embryogenesis) | | 8% decreased in tumor for intermediate grade |
| EXT1 | 8q24.11 | exostosin glycosyltransferase It is a putative tumor suppressor protein, involved in glycosaminoglycan biosynthesis, signal transduction, negative regulation of cell cycle, as well as skeletal development. | 0.6 | 5% decreased in tumor, adjacent and distant histologically normal prostate tissue. |
| SPAG4 | 20q11.21 | sperm associated antigen 4 Structural molecule activity, Spermatogenesis. | 2.1 | 9% increased in tumor, 8% in adjacent and 12% distant histologically normal prostate tissue |

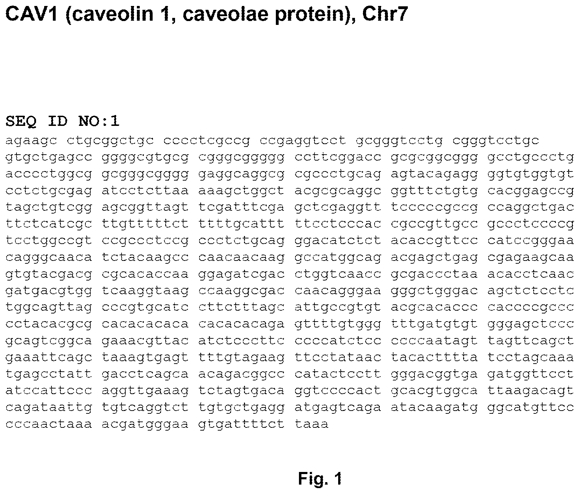

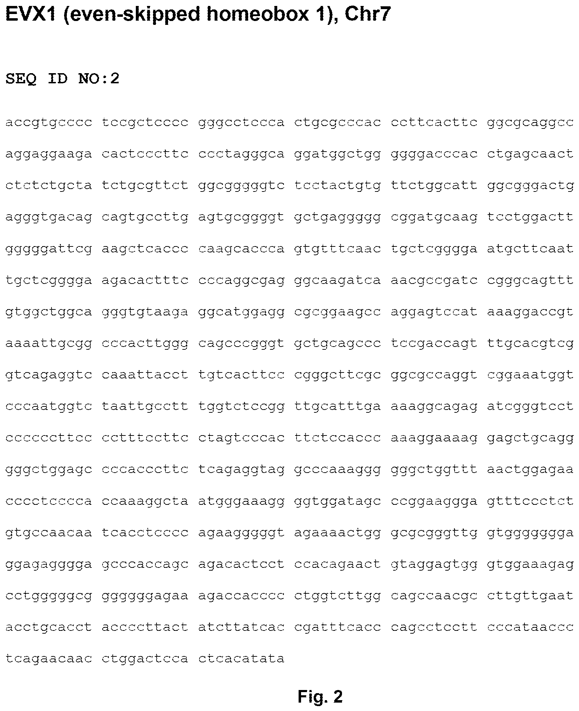

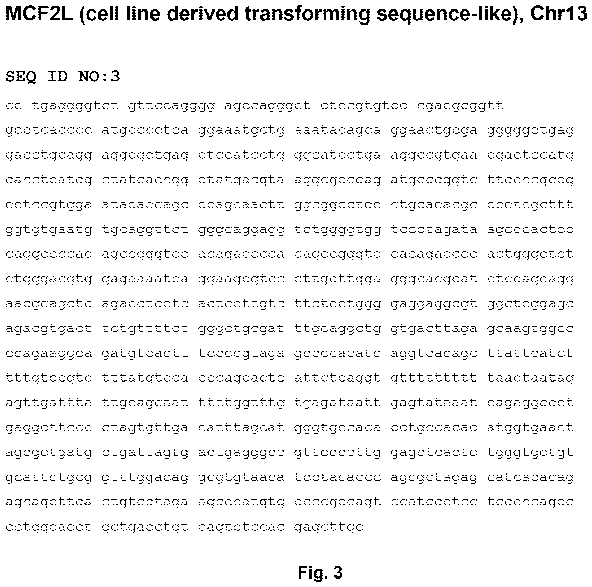

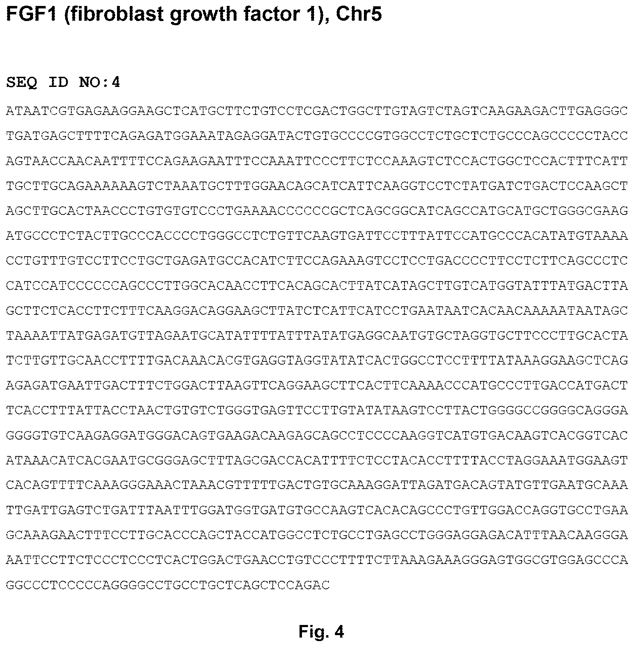

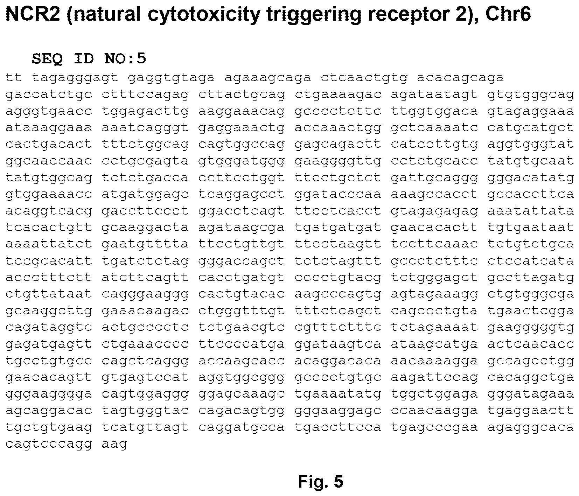

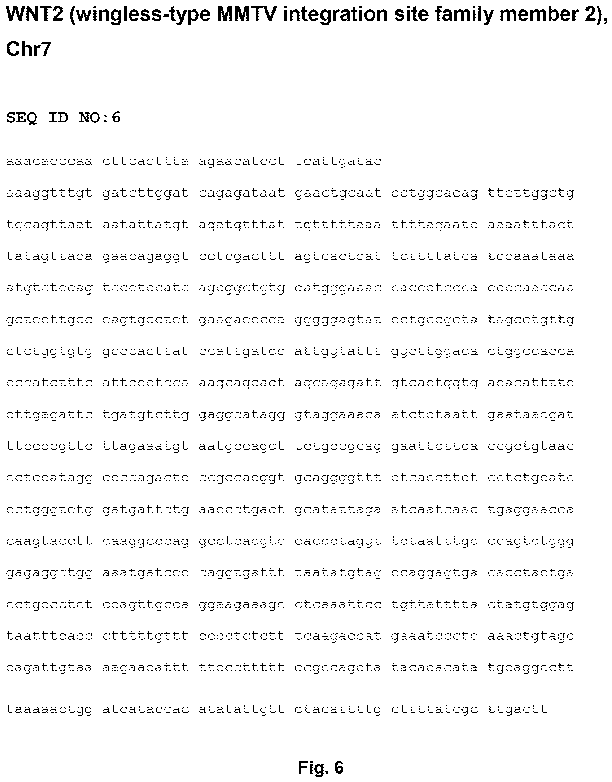

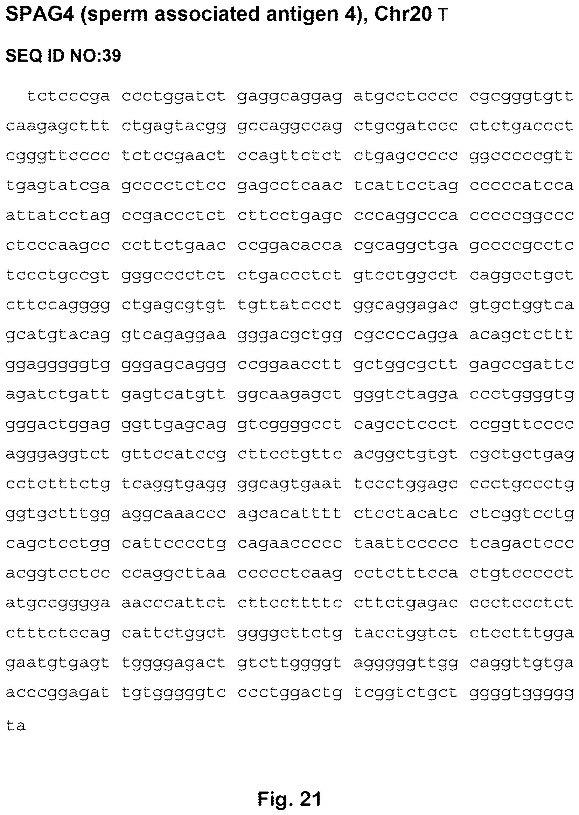

By "gene loci" or "target region", we mean the gene regions described in Figs. 1-6 and 20-21. These are the gene regions in which we correlated either hypermethylation or hypomethylation with a prostate cancer field defect. Fig. 12 describes preferred primer sequences for determining methylation perturbations in these selected target regions. Figs. 12 and 25 describes preferred primer sequences for determining methylation perturbations in these selected target regions. The invention employs at least one target region selected from the group consisting of SEQ ID NO: 18 and 39.

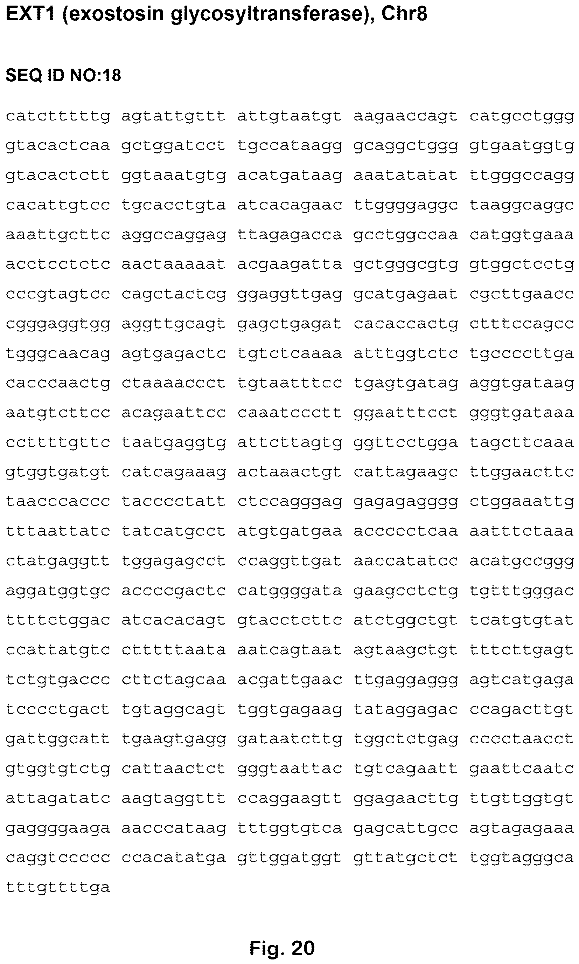

By "gene loci" or "target region", we mean the gene regions described in Figs. 20-21. These are the gene regions in which we correlated either hypermethylation or hypomethylation with a prostate cancer field defect. Fig. 25 describes preferred primer sequences for determining methylation perturbations in these selected target regions. The invention employs at least one target region selected from the group consisting of SEQ ID NO: 18 and 39.

### Embodiments of the Present Invention

In one embodiment, one can diagnose prostate cancer in a human subject by detecting a prostate cancer field defect in histologically normal tissue biopsy specimens taken from men who may have prostate cancer. Based on the results of the detection methods described herein, the subject may be diagnosed with prostate cancer and/or treated for prostate cancer via conventional therapies. It is an advantage of the present invention that fewer biopsies are needed for the detection of prostate cancer. In a preferred embodiment, the presence of prostate cancer field defect can be detected based on only 1-2 core biopsy specimens taken from anywhere in the prostate. Preferably, one would examine one, two, three, four, five, six, seven or eight targets disclosed in Table 1. In addition, in individuals who have had a negative biopsy but whose PSAs continue to rise, analysis of the previously obtained specimens for methylation status in the target regions will direct whether additional evaluation needs to be performed. For example, if the methylation status in any of the target regions is abnormal, a more intensive biopsy set requiring anesthesia would be performed. If not, the patient can be reassured.

In one typical embodiment, the invention is performed on prostate tissue samples obtained via standard transrectal ultrasound and biopsy protocols using an 18 gauge needle (Brooks et al. (2010) J.Natl.Med.Assoc. 102(5), 423-429). In another embodiment, the invention is performed on prostate tissues obtained from paraffin blocks of prostate biopsy samples that have already been obtained and examined.

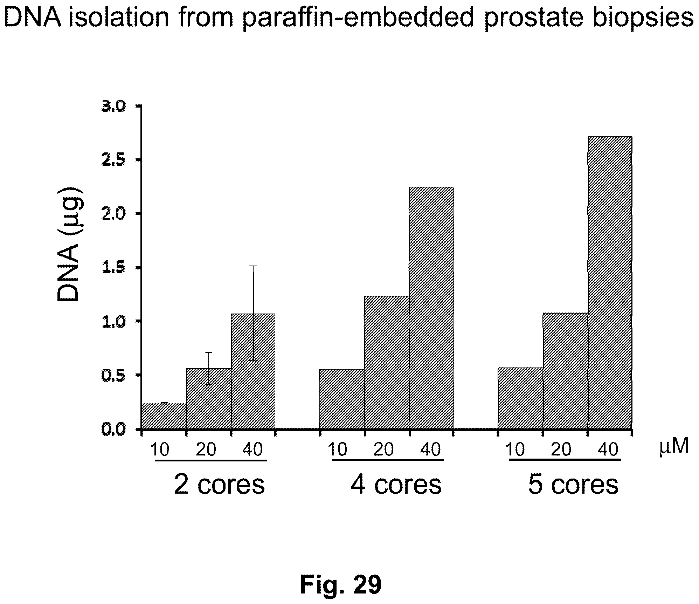

To examine the methylation status of the target regions, one would typically perform the invention on genomic DNA obtained from the tissue samples. The purified genomic DNA is then typically subject to sodium bisulfite modification. We present data demonstrating the ability to obtain enough DNA for analysis using prostate tissue either fresh or paraffin-embedded (See Fig. 29).

In general, bisulfite modified DNA is subjected to PCR reaction containing a single or multiple pair(s) of primers and probes at specific gene loci of at least one of the CAV1, EVX1, MCF2L, FGF1, WNT2, NCR2, EXT1 and SPAG4 loci detailed in Figs. 1-6 and 20-21. The DNA amplification and methylation quantification will be evaluated in one or multiple tubes included as part of a kit. In one embodiment, one would then subject the bisulfite DNA to Methylation-Specific- Quantitative PCR (MS-QPCR) such as MethyLight (WO 00/70090) or HeavyMethyl WO 02/072880). A typical kit for use in the Mehtylight assay of this embodiment would contain primers and probes of target regions detailed in Figs. 1-6, and 20-21, and wild type reference gene primers such as Beta-Actin, PCR buffer, dNTP, MgCl₂, polymerase, positive and negative methylation controls and a dilution reference. Also disclosed is the amplification product described above. In a typical embodiment, the DNA targets are bisulfate-modified DNA. In another typical embodiment, the amplification product comprises the amplification product of 2, 3, 4, 5, 6, 7, or 8 of the targets combined in a vessel, such as a tube or well. Preferably, the DNA amplification product is at least 90% target DNA, most preferably 95% or 99%.

Also disclosed is a combination of the bisulfite-treated DNA described above and materials useful to determine methylation status.

In another embodiment, one would subject the bisulfite DNA to PCR amplification to amplify at least one of the target regions detailed in Figs. 1-6 and 20-21 where at least one of SEQ ID NO: 18 and 39 is amplified from. The PCR products would be subject to pyrosequencing for detection of methylation. The kit for this assay would contain at least one pair of primers for target regions detailed in Figs. 1-6 and 20-21, where at least one pair allowing amplification from SEQ ID NO: 18 or 29 is present, either forward or reverse primer is biotinylated, PCR buffer, dNTPs, MgCl₂, Taq polymerase for bisulfite DNA amplification. A sequencing primer and controls, which typically include positive and negative methylation controls and a dilution reference are typically also included.

In another embodiment, bisulfite treated DNA (initial PCR amplification is needed if bisulfited DNA is less than 20 ng) is subjected to an Invader® assay to detect changes in methylation. The Invader® assay entails the use of Invader® chemistry (Hologic Inc.; invaderchemistry.com; Day, S., and Mast, A. Invader assay, 2004; Chapter in Encyclopedia of Diagnostic Genomics and Proteomics. Marcel Dekker, Inc., U.S. Patent Nos. 7,011,944; 6,913,881; 6,875,572 and 6,872,816). In the Invader® assay, one would use a structure-specific flap endonuclease (FEN) to cleave a three-dimensional complex formed by hybridization of C/T specific overlapping oligonucleotides to target DNA containing a CG site.

The kit for this assay would typically contain the primers and probes of single or multiple target regions detailed in Figs. 1-6 and 20-21, where at least one pair allowing amplification from SEQ ID NO: 18 or 39 is present and controls, which typically include a reference gene such as Beta-Actin, positive and negative methylation controls and a dilution reference.

In another embodiment, the PCR products are purified, denatured to single-strand and annealed to a sequencing primer for methylation quantification by pyrosequencing at the specific gene loci of at least one of the loci described above.

In all embodiments, one would examine the amplification products for a significant change in methylation pattern. One may examine several criteria to evaluate significant change. For example, a finding of ± 50% of the fold-change listed in Table 1 in methylation values of at least one target region selected from the group consisting of SEQ ID NO: 18 and 39 would indicate the presence of a prostate cancer field effect. CAV1, EVX1, MCF2L, FGF1, WNT2 and NCR2 may be additionally employed. Significant change can also be any statistically meaningful change in methylation pattern relative to normal tissue from men with no history of prostate cancer. For example, significant change may be characterized by a p value less than 0.05. As described below, one may wish to use pyrosequencing as a quantitation method and evaluate the sample for the pyrosequencing percentage, as indicated in Table 1.

One may also wish to examine the change in methylation at specific CpG islands. (The Example below discloses specific characterization of CpG islands for the eight target regions.) Preferably, one would determine the methylation status of two, three, four, five, six, seven or eight of the gene loci detailed in Figs. 1-6 and 20 - 21.

As described above, there are many techniques for measuring DNA methylation. For example, one can use Methylation-Specific- Quantitative PCR (MS-QPCR) or to measure DNA Methylation. (See: Eads C.A., MethyLight: a high-throughput assay to measure DNA methylation. Nucleic Acids Res. 2000 Apr 15; 28(8):E32; 2. Darst R.P., Bisulfite sequencing of DNA. Curr Protoc Mol Biol. 2010 Jul; Chapter 7:Unit 7.9.1-17, and Cottrell S.E., et al., A real-time PCR assay for DNA-methylation using methylation specific blockers, Nucleic Acids Res. 2004; 32(1): e10.).

The Examples focus on a preferred method, but one of skill in the art would understand that other methods would be suitable. One simply needs to evaluate the methylation status of CpG islands within the target regions. Examples 1 and 2 below disclose methylation changes at specific CG rich regions, and we anticipate seeing similar changes in adjacent CpG islands not necessarily measured in Examples 1 and 2. Any change in CpG island methylation at one or multiple CG dinucleotides within this island, is considered a positive marker for prostate cancer field defect. One may wish to start with the expanded regions disclosed in Example 3 below.

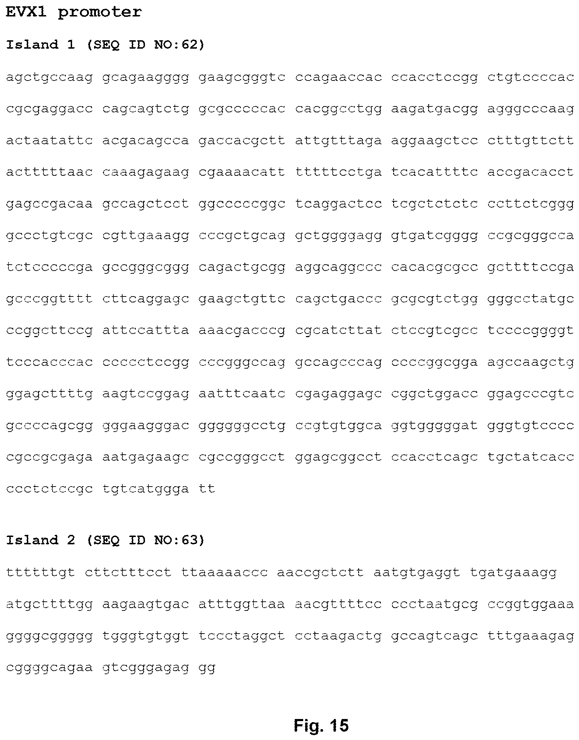

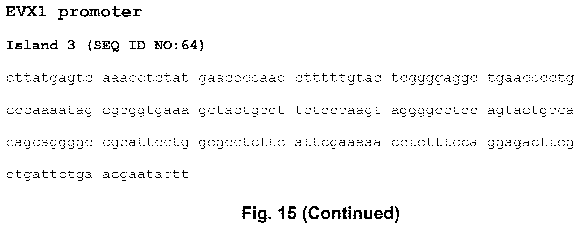

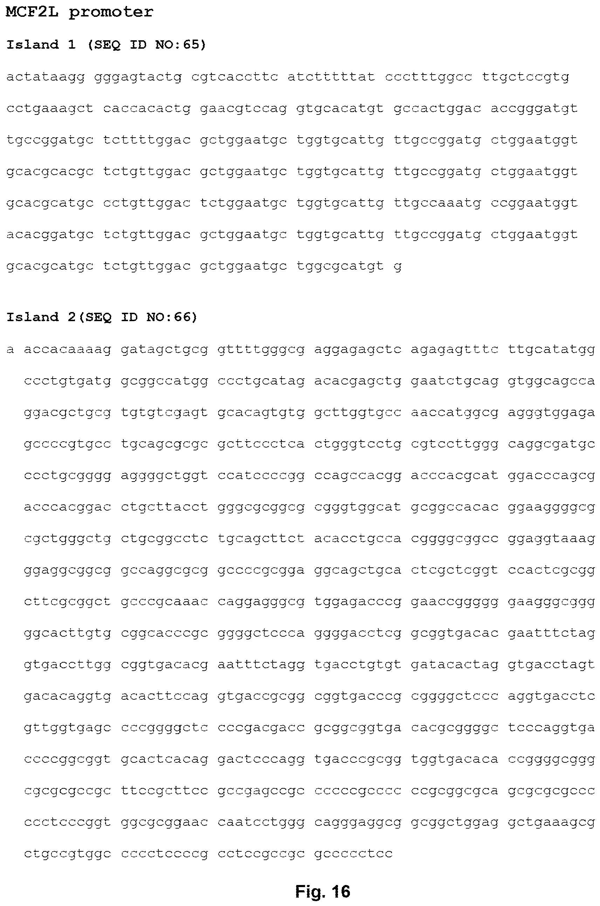

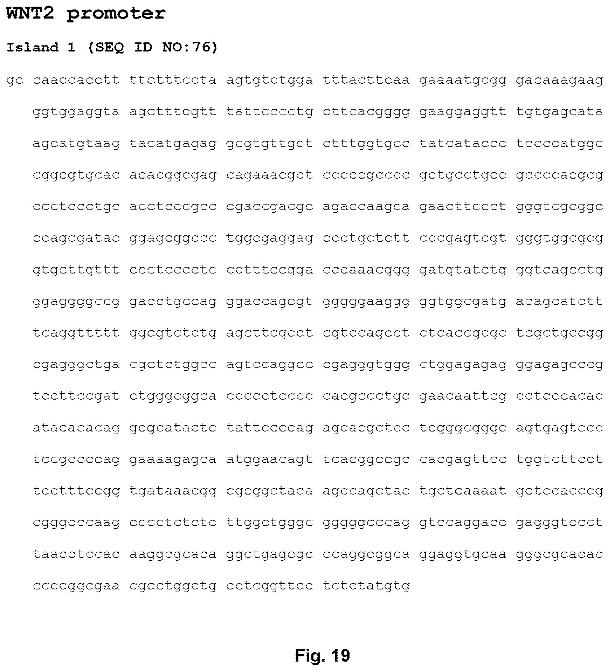

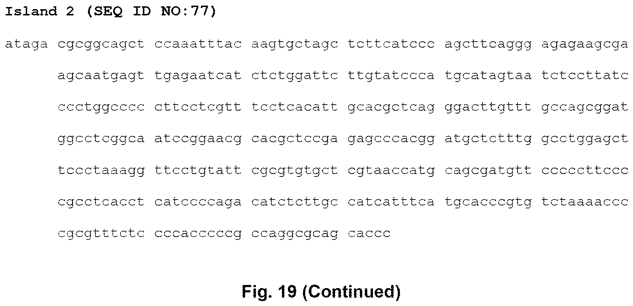

Preferably, one primer within each set of primers is biotinylated, and the biotinylated PCR products are purified, or captured, with Streptavidin sepharose beads. In a preferred embodiment, one would use the primers detailed in Figs. 12-25.

Preferably, the methylation is quantified with PyroMark™MD Pyrosequencing System (Qiagen) using PyroPyroMark® Gold Q96 Reagents (Qiagen, Cat# 972804) (QIAGEN PyroMark Gold Q96 Reagents Handbook 08/2009, (36-38)). Other approaches for methylation quantification include, for example, methylation specific QPCR or quantitative bisulfite sequencing of methylation.

It is an advantage of the present invention that markers for prostate cancer can be detected noninvasively in bodily fluids, such as urine or semen. The bodily fluid screening method currently used is based on PSA levels in serum and has very poor specificity. Biopsies are more specific, but can produce significant clinical complications, including infection, bleeding and urinary retention. Therefore, in one preferred embodiment of the present invention, the methylation status of the target regions is determined from a urine sample.

In another embodiment, the present invention is a method of identifying biomarkers whose DNA methylation changes associate with high grade PCa, using the protocol described above and in the Examples below. By "high grade", we mean PCa with a Gleason Score 8-10 and a tumor volume of 25-80%. For example, a finding of ± 50% of the fold-change in methylation values of at least one gene loci selected from WNT2 and NCR2 would indicate the presence of a high grade PCa field effect. Additional biomarkers for high grade PCa may be identified using the protocol described above and in the Examples below and may also be included in kits.

Generally, patient urine can be obtained, spun and the cell pellet utilized for DNA extraction using protocols as published (Yoshida et al., International Journal of Cancer, n/a-n/a; Mehrotra et al., (2008) Prostate 68, 152-160). One may wish to use DNA methylation urine-based screen for PCa disclosed below in Example 4. One would then analyze the genomic DNA samples as described above for solid tissue samples. Presence of methylation changes correlating to field effect diagnosis would be analyzed in the same manner as described above.

Generally, when pyrosequencing primers (such as the preferred primers in Fig. 12) are used, significant methylation changes of at least one of the eight target regions would indicate a prostate cancer field defect. In various embodiments, significant change is indicated by a value of at least ± 50% of the pyrosequencing percentages shown in Table 1 or ± 50% of the fold-level change in Table 1 or a p<0.05 change in specific CpG island methylation patterns.

In a second embodiment, when pyrosequencing primers (such as the preferred primers in Figs. 12 or 25) are used, significant methylation changes of at least one of the two target regions according to SEQ ID NOs:18 and 39 would indicate a prostate cancer field defect. In various embodiments, significant change is indicated by a value of at least ± 50% of the pyrosequencing percentages shown in Table 1 or ± 50% of the fold-level change in Table 1 or a p<0.05 change in specific CpG island methylation patterns.

In a third embodiment, when pyrosequencing primers (such as the preferred primers in Fig. 12 and/or Fig. 25) are used, significant methylation changes of at least one of the eight target regions according to SEQ ID NOs:1-6, 18 and 39 would indicate a prostate cancer field defect. In various embodiments, significant change is indicated by a value of at least ± 50% of the pyrosequencing percentages shown in Table 1 or ± 50% of the fold-level change in Table 1 or a p<0.05 change in specific CpG island methylation patterns.

It is another advantage of the present invention that changes in methylation levels of the disclosed markers for prostate cancer can be detected in histologically normal prostate tissue or bodily fluid from men with no history of prostate cancer.

Yet another embodiment of the invention recognizes that the markers can also be used to monitor changes to the prostate as a result of future drug treatments that modify methylation or to assess the clinical severity of an at-risk or cancer patient.

Also referred to is the use evaluation of methylation status of at least one of the eight target regions for the diagnosis of other cancers, such as breast or colon cancer.

In another embodiment, the present invention is a method of amplifying at least one of target DNA sequence to diagnose prostate cancer in a subject comprising the steps of
(a) providing a reaction mixture comprising a double-stranded bisulfite modified target DNA of a subject and (i) at least one pair of primers selected from the group designed to amplify at least one gene selected from the group consisting of SEQ ID NO: 18 and SEQ ID NO: 39, wherein the primer pair comprises a first and a second primer that are complementary to the target DNA sequence, (ii) a polymerase and (iii) a plurality of free nucleotides comprising adenine, thymine, cytosine and guanine;
(b) heating the reaction mixture to a first predetermined temperature for a first predetermined time to separate the strands of the target DNA from each other;
(c) cooling the reaction mixture to a second predetermined temperature for a second predetermined time under conditions to allow the first and second primers to hybridize with their complementary sequences on the target DNA and to allow the polymerase to extend the primers; and
(d) repeating steps (b) and (c) at least 10 times, wherein increased methylation, relative to tissue from a second human subject who does not have prostate cancer, indicates the presence of prostate cancer or a prostate cancer field defect.

In one embodiment, the primers are methylated. In another embodiment, the primers are not methylated. In one embodiment, one would use a primer pair designed to amplify one target. In another embodiment, one would use primer pairs designed to amplify 2, 3, 4, 5, 6, 7, or 8 target regions.

### Kits

In another embodiment, the present invention also provided is use of a kit for prostate cancer diagnosis or prostate cancer field defect detection, the kit comprising at least a set of primers, wherein the primer set consists of a forward primer and a reverse primer designed to amplify a target region selected from the group consisting of SEQ ID NOs: 18 and 39, and components essential for DNA amplification. Typically, the kit comprises at least a set of primers, wherein the primers preferably comprise forward and reverse primers designed to amplify a target region selected from the group consisting of CAV1, EVX1, MCF2L, FGF1, NCR2, WNT2, EXT1 and SPAG4 target (SEQ ID NOs: 1-6, 18 and 39), or selected from the group consisting of SEQ ID NOs: 61-77 and 94-97, and other components essential for DNA amplification, preferably, polymerase, dNTP, buffer and a magnesium salt which can release Mg²⁺. Typically, one can use MgCl₂ or MgSO₄. In other embodiments, the kit used comprises primers designed to amplify two, three, four, five, six, seven or eight targets.

In one embodiment, the primers preferably comprise a forward primer selected from the group consisting of SEQ ID NOs:43, 46, 49, 52, 55 and 58, and a reverse primer selected from the group consisting of SEQ ID NOs:44, 47, 50, 53, 56 and 59, and other components essential for DNA amplification, preferably, polymerase, dNTP, buffer and a Magnesium salt which can release Mg²⁺. Typically, one can use MgCl₂ or MgSO₄.

In a second embodiment, the aforementioned kit used comprises an alternative set of primers, wherein the primers preferably comprise a forward primer selected from the group consisting of SEQ ID NOs:88 and 91, and a reverse primer selected from the group consisting of SEQ ID NOs:89 and 92.

In a third embodiment, the aforementioned use of a kit comprising a combined set of primers, wherein the primers preferably comprise a forward primer selected from the group consisting of SEQ ID NOs: 43, 46, 49, 52, 55, 58, 88 and 91, and a reverse primer selected from the group consisting of SEQ ID NOs: 44, 47, 50, 53, 56, 59, 89 and 92 wherein at least a forward primer selected from the group consisting of SEQ ID No: 88 and 91, and a reverse primer selected from the group consisting of SEQ ID No: 89 and 92 is present.

In one preferred embodiment, the kit used further comprises FAM or Hex fluorophore-labled methylation and unmethylation-specific probes and is suitable for a closed tube assay for MS-QPCR. In another preferred embodiment, the kit used further comprises sequencing primers and is suitable for bisulfite pyrosequencing-based assay. Preferably, the sequencing primers are selected from the group consisting of SEQ ID NOs: 45, 48, 51, 54, 57 and 60. Even more preferably, the kit used further comprises Streptavidin sepharose beads, enzyme mixture, substrate mixture and dinucleotides.

In a second preferred embodiment, the kit used further comprises sequencing primers selected from the group consisting of SEQ ID NOs: 90 and 93.

In a third preferred embodiment, the kit used further comprises sequencing primers selected from the group consisting of SEQ ID NOs: 45, 48, 51, 54, 57, 60, 90 and 93.

In another embodiment, the kit used comprises components for an Invader® assay to detect changes in methylation. The Invader® assay entails the use of Invader® chemistry (Hologic Inc.) which is composed of two simultaneous isothermal reactions. A primary reaction specifically and accurately detects single-base pair changes measuring methylation. A second reaction is used for signal amplification and result readout.

### EXAMPLES

### Example 1

Prostate cancer (PCa) is typically found as a multifocal disease suggesting the potential for molecular defects within the morphologically normal tissue. In Example 1, the inventors compared non-tumor associated (NTA) prostate to histologically indistinguishable tumor-associated (TA) prostate tissues and detected a distinct profile of DNA methylation alterations (0.2%) using genome-wide DNA arrays. Hypomethylation (87%) occurred more frequently than hypermethylation (13%). Analysis of TA tissues adjacent and distant from tumor foci revealed a persistence of this methylation defect. Further evaluation and validation of six loci distinguished TA from NTA patients. Still further evaluation and validation of two additional loci distinguished TA from NTA patients. The inventors found a subset of markers which were solely associated with the presence of high grade disease. These findings demonstrate a widespread methylation defect occurs in the peripheral prostate tissues of men with PCa that may be utilized to identify the presence of the disease.

### Introduction

'Field cancerization', 'field effect' or 'field defect' were terms first utilized in head and neck tumors to describe an increased frequency of cancer development found outside the visible boundaries of the primary tumor¹. These genetically or epigenetically compromised cells in histologically normal appearing tissues have the potential to give rise to not only multifocal tumors, but additional cancers after therapy. Although described in colorectal, bladder and esophageal cancer (Jothy et al. (1996) Field effect of human colon carcinoma on normal mucosa: relevance of carcinoembryonic antigen expression. Tumour Biol 17, 7; Takahashi, T., et al. (1998) Clonal and Chronological Genetic Analysis of Multifocal Cancers of the Bladder and Upper Urinary Tract, Cancer Research 58, 5835-5841; Miyazato, et al. (1999) Microsatellite instability in double cancers of the esophagus and head and neck, Diseases of the Esophagus 12, 132-136; Ushijima, T. (2007) Epigenetic Field for Cancerization, Journal of Biochemistry and Molecular Biology, Vol. 40, No. 2, March 2007, pp. 142-150 40, 9), a field effect has not been clearly defined for prostate cancer (PCa). Features suggesting the presence of a field effect in PCa include regional multifocality at diagnosis, as well as the increased incidence with aging (Eastham, J.A., et al. (2007) Prognostic Significance of Location of Positive Margins in Radical Prostatectomy Specimens, Urology 70, 965-969). Defining an epigenetic field defect associated with PCa would have important clinical ramifications with regard to recurrence and recent interest in focal ablative therapies (Mouraviev, V., et al. Prostate cancer laterality as a rationale of focal ablative therapy for the treatment of clinically localized prostate cancer, Cancer 110, 906-910 (2007)).

PCa development and progression is driven by the interplay of genetic and epigenetic changes (Schulz, W.A. & Hoffmann, M.J. Epigenetic mechanisms in the biology of prostate cancer, Semin Cancer Biol 19, 172-180 (2009)). One important epigenetic process is the reversible methylation of cytosine at CpG dinucleotides, a sequence underrepresented in the genome except at CpG islands (Brid, A. DNA methylation patterns and epigenetic memory, Genes Dev 16, 16 (2002)). DNA methylation regulates gene expression and participates in the nuclear organization of higher organisms. Alterations in DNA methylation are a hallmark of cancer. Typically, adjacent histologically normal tissues are the standard against which many genomic and epigenetic alterations in cancers are identified. In light of the relevance of a potential field defect to both molecular and clinical studies, little is known regarding its distribution and extent in PCa. In part, this has reflected a limitation of techniques for assessing DNA methylation at specific sequences throughout the genome, as well as a lack of specimens without histological evidence of PCa.

In the Example below, the inventors utilized an immunocapture approach to enrich methylated DNA and combine this with DNA microarrays. During an evaluation of control tissues for genome-wide methylation profiles in cancer, the inventors found marked methylation changes in tumor associated (TA) histologically normal appearing prostate tissues extending across susceptible prostate tissues.

### Results

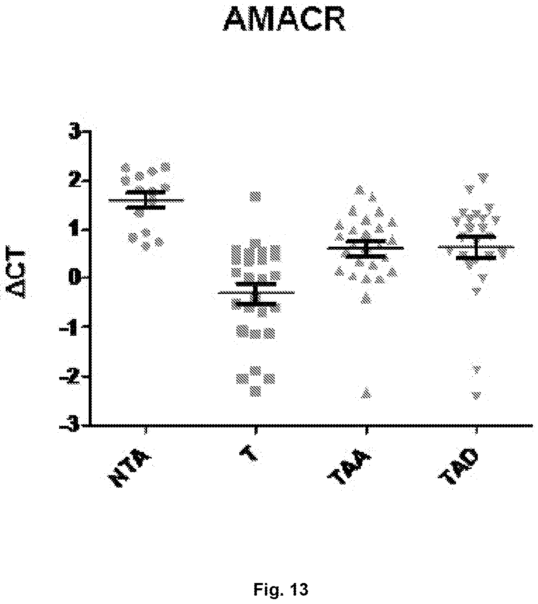

Distinct patterns of DNA methylation define tumor associated (TA) and non-tumor associated (NTA) prostate tissues
As an initial study of the proper controls for cancer analyses, the inventors undertook an analysis of genome-wide methylation changes in histologically normal prostate tissues from men with cancer and compared those to men without cancer. We utilized 385,000 locus arrays based on the Encyclopedia of DNA Elements (ENCODE) 18 sequence that tiles a series of biologically significant regions in the human genome and includes all chromosomes except chromosomes 3 and 17. DNA was initially prepared from four TA and five NTA prostate specimens, digested with restriction enzymes and enriched for methylated DNA by immunoprecipitation (IP) with an antibody against 5-methylcytidine as described (User's, N.S.P.I.i.N. & Guide: DNA Methylation Analysis). Peripheral zone prostate tissues were utilized for these studies as PCa demonstrates a predilection for this region. We carefully evaluated all NTA specimens to confirm the lack of PCa within the prostate by both H&E staining in three dimensions and α-methylacyl-Coa racemase (AMACR) expression (Fig. 13). Furthermore, the proportion of epithelium to stroma was similar between tissue groups. After labeling, differential hybridization and scanning, we used a probe score cut-off of -log₁₀ [p] range 2 - 10 to generate about 1,000 probes for each chromosome and a total of 18,101 probes. We then compared the log₂-ratios at individual probes for TA and NTA tissues to evaluate methylation.

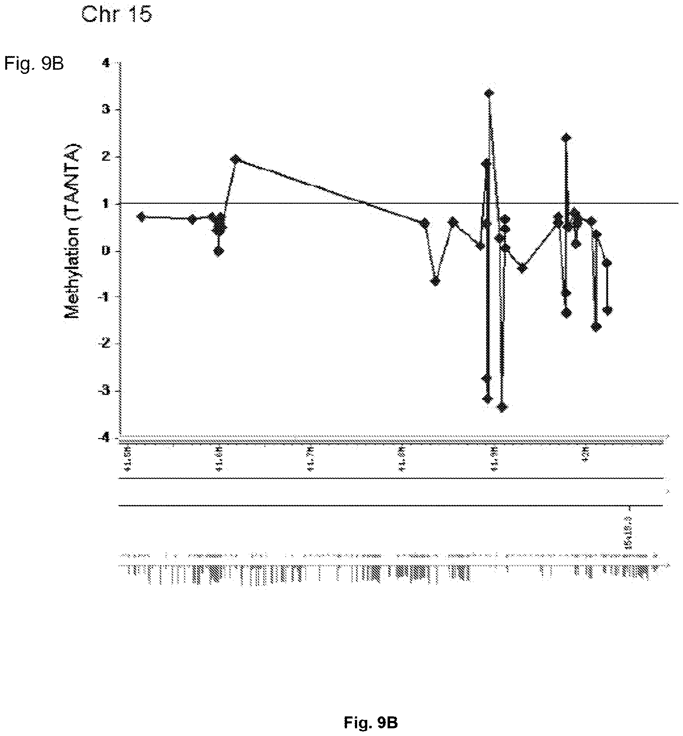

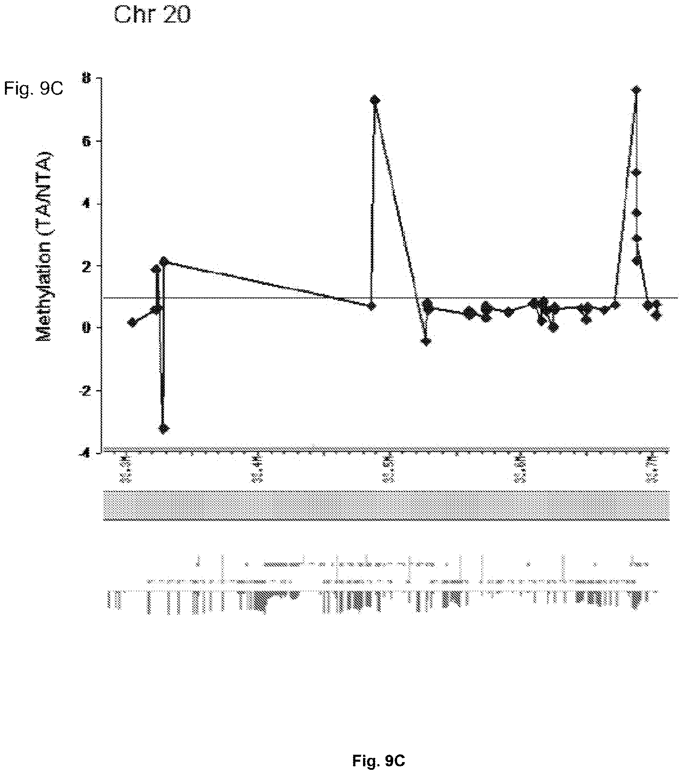

Striking differences in methylation were noted when TA and NTA tissues were compared. With P < 0.05, 615 loci were identified to be differentially methylated in TA tissues, with 537 (87%) hypomethylated and 78 (13%) hypermethylated (Fig. 9A). Chromosome 15 demonstrated the greatest number of differentially methylated loci (1.13%) in TA tissues, followed by chromosome 20 (0.9%), 1 (0.57%) and 9 (0.51%). Across genomic regions specific areas demonstrated either hyper- or hypomethylation (Fig. 9B and Fig. 9C). Fold changes in methylation for TA vs. NTA prostate specimens ranged from 0.02-7.59 (data not shown).

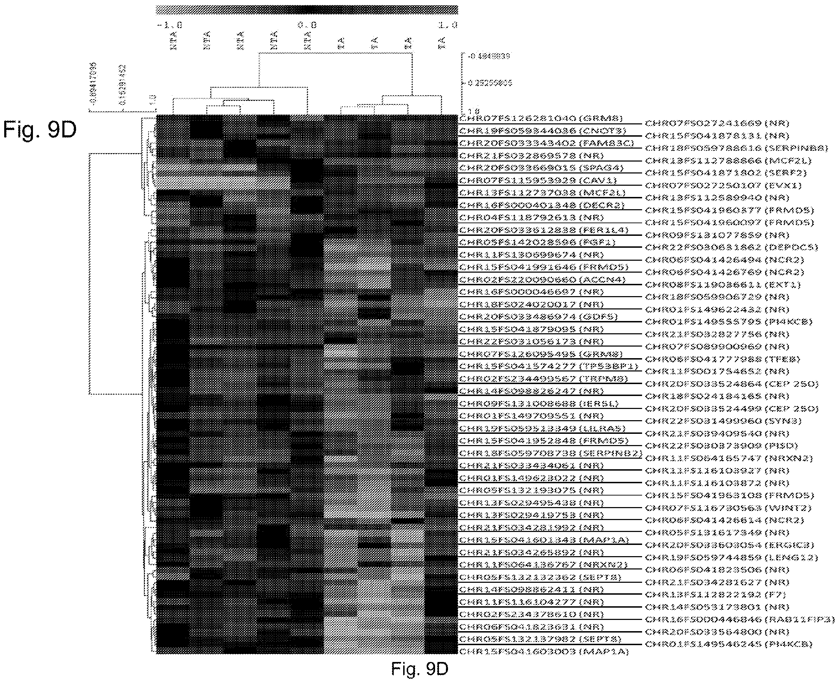

Using more stringent statistical parameters (P < 0.01), the inventors identified 87 loci which showed significantly differential methylation in TA prostates. These loci were subject to unsupervised hierarchical clustering using TMEV software to generate a heat map. This global view of methylation profile clearly distinguishes TA from NTA prostate tissues (Fig. 9D). Among the 87 loci, 69 were hypomethylated and 18 hypermethylated in TA tissues (Table 2). Of these, 49 probes were associated with 38 genes and 38 probes were non-gene related. Accession numbers for these genes are listed in Table 3.

**Table 2 - Location of Differentially Methylated Probes**

| | | Tumor-Associated vs Normal | |
|---|---|---|---|
| Chromosome location | Total Probe No. | Hypomethylation | Hypermethylation |
| 1 | 5 | P14KB (2), NR (3) | |
| 2 | 3 | ACCN4 (1), TRPM8 (1), NR (1) | |
| 4 | 1 | | NR (1) |
| 5 | 5 | SEPT8 (2), FGF1 (1), NR (2) | |
| 6 | 6 | NCR2 (3), TFEB (1), NR (2) | |
| 7 | 7 | WINT2 (1), GRM8 (1), NR (1) | EVX1 (1), GRM8 (1) |
| | | | CAV1 (1), NR (1) |
| 8 | 1 | EXT1 (1) | |
| 9 | 2 | IER5L (1), NR (1) | |
| 11 | 7 | NRXN2 (2), NR (5) | |
| 13 | 6 | F7 (1), NR (2) | MCF2L (2), NR (1) |
| 14 | 3 | NR (3) | |
| 15 | 11 | TP53BP1 (1), MAP1A (2), FRMD5 (3), NR (1) | FRMD5 (2), SERF2 (1), NR (1) |
| 16 | 3 | RAB11FIP3 (1), NR (1) | DECR2 (1) |
| 18 | 5 | SERPINB2 (1), NR (3) | SERPINB8 (1) |
| 19 | 3 | LILRA5 (1), LENG12 (1) | CNOT3 (1) |
| 20 | 8 | GDF5 (1), CEP250 (2), ERGIC3 (1), FER1L4 (1), NR (1) | FAM83C (1), SPAG4 (1) |
| 21 | 7 | NR (6) | NR (1) |
| 22 | 4 | DEPDC5 (1), SYN3 (1), PISD (1), NR (1) | |
| Total | 87 | 69 | 18 |

| | | | |
|---|---|---|---|
| Significant methylated probes between normal and tumor-associated prostate were generated from Methylation array using a cut-off probes score-log10 [p] ranged from 2-10 to generate 18,101 probes in total, and then log2ratio for these probes were compared between TA and NTA , t-test P<0.01. Sixty-nine probes were hypomethylated, 36 probes related to 27 non-gene regions. NR represents not related to any gene. | | | |

**Table 3**

| **Gene Symbol** | **Gene Name** | **Accession #** |
|---|---|---|
| P14KCB | Phosphatidylinosol 4-kinase, catalytic, beta | NM_002651 (SEQ ID NO:7) |
| ACCN4 | Amiloride-sensitive cation channel, pituitary | NM_182847 (SEQ ID NO:8) |
| TRPM8 | Transient receptor potential cation channel, subfamily M, member 8 | NM_024080 (SEQ ID NO:9) |
| SEPT8 | Septin | AF440762 (SEQ ID NO:10) |
| FGF1 | Fibroblast growth factor 1 (acidic) | NM_000800 (SEQ ID NO:11) |
| NCR2 | Natural cytotoxicity triggering receptor 2 | AJ010100 (SEQ ID NO:12) |
| TFEB | Transcription factor EB | NM_007162 (SEQ ID NO:13) |
| EVX1 | Even-skipped homeobox 1 | NM_001989 (SEQ ID NO:14) |
| CAV1 | Caveolin 1 | NG_012051.1 (SEQ ID NO:15) |
| WNT2 | Wingless-type MMTV integration site family member 2 | BC078170 (SEQ ID NO:16) |
| GRM8 | Glutamate receptor, metabotropic 8 | NM_000845 (SEQ ID NO:17) |
| EXT1 | Exosloses (multiple) 1 | BC001174 (SEQ ID NO:18) |
| IER5L | Immediate early response 5-like | NM_203434 (SEQ ID NO:19) |
| NRXN2 | Neurexin 2 | NM_138734 (SEQ ID NO:20) |
| MCF2L | Cell line derived transforming sequence-like | NM_024979 (SEQ ID NO:21) |
| F7 | Coagulation factor VII | NM_019616 (SEQ ID NO:22) |
| TP53BP1 | Tumor protein p53 binding protein 1 | NM_005657 (SEQ ID NO:23) |
| MAP1A | Microtubule-associated protein 1A | NM_002373 (SEQ ID NO:24) |
| SERF2 | Small EDRK-rich factor 2 | BC015491 (SEQ ID NO:25) |
| FRMD5 | FERM domain containing 5 | NM_032892 (SEQ ID NO:26) |
| DECR2 | 2,4-dienoyl CoA reductase 2, peroxisomal | AK128012 (SEQ ID NO:27) |
| RAB11FIP3 | RAB11 family interacting protein 3 (class III) | NM_014700 (SEQ ID NO:28) |
| SERPINB2 | Serpin peptidase inhibitor, clade B (ovalbumin), member 2 | NM_002575 (SEQ ID NO:29) |
| SERPINB8 | Serpin peptidase inhibitor, clade B (ovalbumin), member 8 | BC034528 (SEQ ID NO:30) |
| CNOT3 | CCR4-NOT transcription complex, subunit 3 | BC016474 (SEQ ID NO:31) |
| LILRA5 | Leukocyte immunoglobulin-like receptor, subfamily A (with TM domain), member 5 | NM_181985 (SEQ ID NO:32) |
| LENG12 | Leukocyte receptor cluster (LRC) member 12 | NM_033206 (SEQ ID NO:33) |
| FAM83C | Family with sequence similarity 83, member C | NM_178468 (SEQ ID NO:34) |
| GDF5 | Growth differentiation factor 5 | NM_000557 (SEQ ID NO:35) |
| CEP250 | Centrosomal protein | AF022655 (SEQ ID NO:36) |
| ERGIC3 | ERGIC and golgi 3 | NM_015966 (SEQ ID NO:37) |
| FER1 L4 | Fer-1-like 4 | NR_024377.1 (SEQ ID NO:38) |
| SPAG4 | Sperm associated antigen | NM_003116 (SEQ ID NO:39) |
| PISD | Phosphatetidylserine decarboxylase | CR456540 (SEQ ID NO:40) |
| DEPDC5 | DEP domain containing 5 | AJ698951 (SEQ ID NO:41) |
| SYN3 | Synapsin III | NM_003490 (SEQ ID NO:42) |

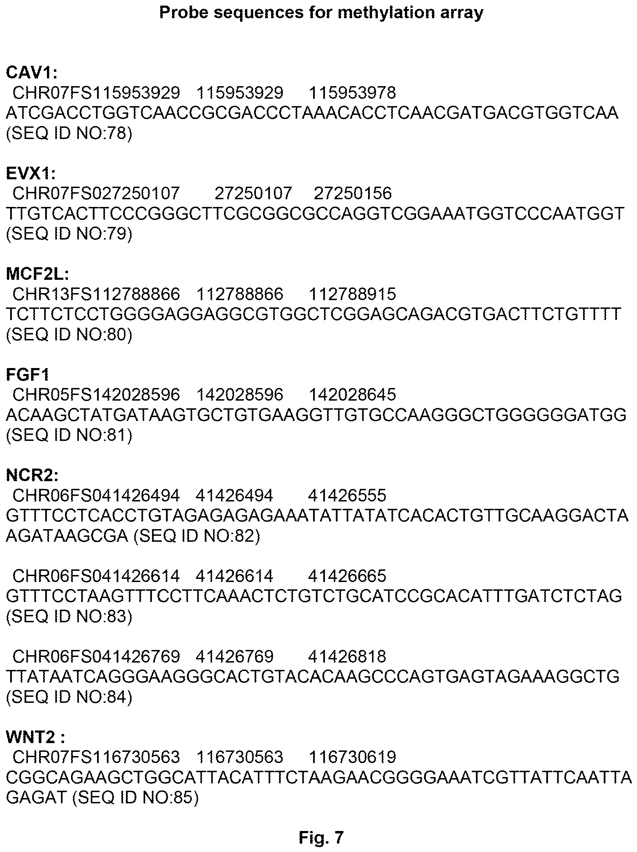

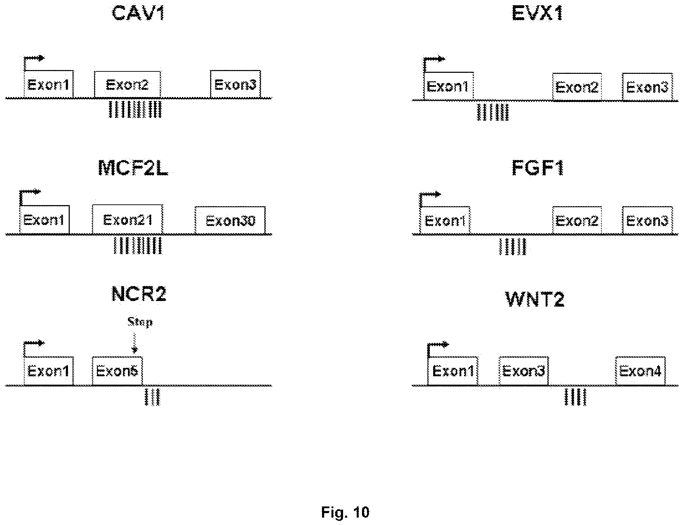

A subset of the 20 genes were chosen for further evaluation, based on genomic location, putative biological function, extent of methylation and primer success in a separate validation using a set of 24 TA and NTA prostate specimens. Quantitative Pyrosequencing was employed to allow a more accurate evaluation of the extent of DNA methylation ^{11,12}. Internal controls for the adequacy of bisulfite conversion were performed. Six loci, which were associated with the genes CAV1, EVX1, MCF2L, FGF1, NCR2 and WNT2, showed significant methylation changes (P < 0.05). The three loci associated with CAV1, EVX and MCF2L were hypermethylated and the three loci associated with FGF1, NCR2 and WNT2 were hypomethylated. The location of the probes and CG's assessed by Quantitative Pyrosequencing are shown in Figs. 10 and 12. The six loci in pyrosequencing are close or overlap the methylation array regions but sequences are different. The sequences listed in Fig 1-6 have covered both array region (Fig 7) and pyrosequencing regions. These data demonstrate that TA tissues have a methylation profile distinct from men without cancer (NTA) and that these changes alter specific regions of the genome.

### Identification of a widespread methylation field defect in the peripheral prostate.

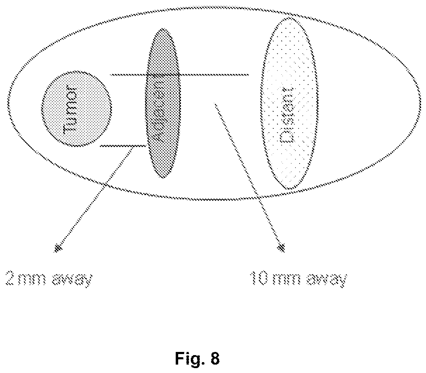

Preferential alteration in tissues adjacent to PCa tumor foci, i.e., field defect, suggests a peritumoral response. To evaluate whether tissues adjacent to PCa tumor foci are preferentially altered, the extent of field defect was assessed in 26 additional histologically normal tissues by looking at the methylation status of these six differentially methylated markers. The inventors micro-dissected normal tissues adjacent (TAA, 2 mm) and distant (TAD, >10 mm) from the main tumor focus for each of the specimens (Fig. 8). Histological 3-dimensional H&E staining and AMACR expression determined by qPCR were applied to rule out any contamination by tumor cells or the presence of high grade prostatic intraepithelial neoplasia (HGPIN), a putative cancer precursor (Ayala, A.G. & Ro, J.Y. Prostatic Intraepithelial Neoplasia: Recent Advances, Archives of Pathology & Laboratory Medicine 131, 1257-1266 (2007)). Increased AMACR expression was found in 2 NTA and 3 TA tissues that were subsequently excluded from further analysis (Fig 13).

When compared to NTA tissues, hypermethylation of probes associated with CAV1, EVX1, MCF2L and hypomethylation of FGF1 demonstrated significant changes in both TAA, as well as TAD tissues (Fig. 11A-D and Table 4). Notably, there was no difference in the extent of methylation seen at different distances from the tumor when TAA and TAD tissue sets were compared. Significant methylation changes were also seen in tumor samples when compared to NTA tissues for CAV1, EVX1, MCF2L, NCR2 and WNT2, revealing a persistence of these changes in the associated cancer. These data indicate that the epigenetic field defect in the prostate is widespread and not solely localized to the immediate peritumor environment.

**Table 4 - Methylation Percentage Of All Analyzed CpGs For Each Gene**

| | **CAV1** | | | **EVX1** | | | **MCF2L** | | | **FGF1** | | | **NCR2¹** | | | **WNT2¹** | | |
|---|---|---|---|---|---|---|---|---|---|---|---|---|---|---|---|---|---|---|
| | **NTA** | **TAA** | **TAD** | **NTA** | **TAA** | **TAD** | **NTA** | **TAA** | **TAD** | **NTA** | **TAA** | **TAD** | **NTA** | **TAA** | **TAD** | **NTA** | **TAA** | **TAD** |
| **CG1** | **4.5** | **8.8*** | **9.6*** | **30.5** | **38.8*** | **32.6** | **80.2** | **85.2*** | **85.3*** | **80.4** | **70.7*** | **70.8*** | **54.3** | **50.8** | **52.1** | **95.4** | **89.8*** | **89.8*** |
| **CG2** | **14.6** | **22.4*** | **21.3*** | **28.2** | **36.9*** | **29.9** | **77.0** | **85.3*** | **85.1** | **71.7** | **60.7*** | **59.8*** | **30.5** | **30.6** | **30.9** | **94.9** | **91.0*** | **91.5*** |
| **CG3** | **17.8** | **27.7*** | **25.8*** | **22.7** | **30.8*** | **27.8*** | **96.3** | **97.4** | **96.5** | **71.2** | **60.2*** | **60.9*** | **74.7** | **68.6*** | **70.7** | **100** | **99.5** | **100** |
| **CG4** | **13.8** | **24.3*** | **23.0*** | **50.4** | **55.4** | **48.3** | **84.8** | **82.1** | **80.7** | **81.1** | **72.9*** | **71.1*** | | | | **99.8** | **99.5** | **100** |
| **CG5** | **15.3** | **25.0*** | **21.9*** | **46.5** | **51.7** | **47.2** | **79.9** | **86.1** | **87.5** | | | | | | | | | |
| **CG6** | **14.9** | **27.2*** | **26.4*** | **36.7** | **44.8*** | **40.6*** | **75.3** | **81.0** | **82.1** | | | | | | | | | |
| **CG7** | **18.9** | **28.0*** | **26.0** | | | | **89.6** | **94.3** | **93.6** | | | | | | | | | |
| **CG8** | **8.25** | **15.4*** | **14.7*** | | | | **57.8** | **57.2** | **55.8** | | | | | | | | | |
| **CG9** | **15.8** | **22.7** | **19.5** | | | | **39.8** | **31.4** | **38.1** | | | | | | | | | |
| **CG10** | **17.9** | **26.7*** | **28.6*** | | | | | | | | | | | | | | | |

| | | | | | | | | | | | | | | | | | | |
|---|---|---|---|---|---|---|---|---|---|---|---|---|---|---|---|---|---|---|
| *P<0.05 ¹ High grade tumor only | | | | | | | | | | | | | | | | | | |

### Specific methylation loci are associated with a high-grade PCa field defect.

An important issue in PCa is the early identification and treatment of lethal high grade PCa. The inventors Analyzed a subset of TA tissues that were associated with either intermediate or high grade cancer using pyrosequencing. When compared to NTA tissues, an analysis of NCR2 and WNT2 demonstrated significant hypermethylation and hypomethylation, respectively, in TA tissues associated with high-grade specimens (Fig. 11E-F). This was not seen in TA tissues associated with intermediate grade PCa.

### Discussion

Research has theorized that a field defect may underlie the development of multifocal cancers (Slaughter D.P., Southwick H.W., Smejkal, W.; Field cancerization in oral stratified squamous epithelium; Clinical implications of multicentric origin, Cancer 6, 6 (1953)). Initial efforts in characterizing this process focused on genetic alterations (Braakhuis, B.J.M., Tabor, M.P., Kummer, J.A., Leemans, C.R. & Brakenhoff, R.H., A Genetic Explanation of Slaughter's Concept of Field Cancerization, Cancer Research 63, 1727-1730 (2003); Garcia, S.B., Park, H.S., Novelli, M. & Wright, N.A. Field cancerization, clonality, and epithelial stem cells: the spread of mutated clones in epithelial sheets, The Journal of Pathology 187, 61-81 (1999)), but more recently epigenetic changes have been proposed as a etiology (Hu, M., et al. Distinct epigenetic changes in the stromal cells of breast cancers, Nat Genet 37, 899-905 (2005); Wolff, E.M., et al., Unique DNA Methylation Patterns Distinguish Noninvasive and Invasive Urothelial Cancers and Establish an Epigenetic Field Defect in Premalignant Tissue, Cancer Research 70, 8169-8178). In the present study, we conclusively demonstrate, using unbiased methylation arrays, that significant changes in DNA methylation occur at specific loci within histologically normal tissues associated with PCa. Furthermore, these changes are widespread and not restricted to the immediate peritumor environment. These changes also permit a clear distinction between tumor associated and non-tumor associated prostate tissue.

To date, epigenetic profiling of tumor-associated histologically normal tissues has not been performed in solid tumors. Our genome-wide assessment of specific loci demonstrates that hypomethylation was seen more commonly than hypermethylation in TA prostate tissues. These changes occurred in 0.2% of the 385,000 loci studied. DNA hypomethylation may occur early in solid tumor carcinogenesis based on its identification in precancerous lesions, including prostatic intraepithelial neoplasia (Feinberg, A.P., Ohlsson, R. & Henikoff, S., The epigenetic progenitor origin of human cancer, Nat Rev Genet 7, 21-33 (2006); Suzuki, K., et al. Global DNA demethylation in gastrointestinal cancer is age dependent and precedes genomic damage, Cancer Cell 9, 199-207 (2006)). This may lead to chromatin instability and contribute to the neoplastic phenotype. Our data extend these findings and suggest that epigenetic alterations may precede even the histologic changes identified with these precursor lesions. These DNA methylation changes may reflect diet and other environmental exposures (Richardson, B.C., Role of DNA Methylation in the Regulation of Cell Function: Autoimmunity, Aging and Cancer, The Journal of Nutrition 132, 2401S-2405S (2002); Mathers JC , S.G., Relton CL, Induction of epigenetic alterations by dietary and other environmental factors, Adv Genet. 71, 37 (2010)) and represent a potential avenue for prevention.

Epigenetic alterations limited solely to the immediate peritumor environment suggest a response of the surrounding tissue to the primary cancer. Single gene epigenetic studies have identified these changes in a subset of specimens adjacent to the primary PCa (Mehrotra, J., et al., Quantitative, spatial resolution of the epigenetic field effect in prostate cancer, Prostate 68, 152-160 (2008); Aitchison, A., Warren, A., Neal, D. & Rabbitts, P. RASSF1A promoter methylation is frequently detected in both pre-malignant and non-malignant microdissected prostatic epithelial tissues, Prostate 67, 638-644 (2007); Hanson, J.A., et al., Gene Promoter Methylation in Prostate Tumor-Associated Stromal Cells, J. Natl. Cancer Inst. 98, 255-261 (2006); Henrique, R., et al., Epigenetic heterogeneity of high-grade prostatic intraepithelial neoplasia: clues for clonal progression in prostate carcinogenesis, Mol Cancer Res 4, 1-8 (2006)). In contrast, in the present epigenomic profiling study, we found that these alterations consistently extended to regions distant from tumor foci. In bladder cancer, a disease also characterized by multifocality and recurrence, there is no dependence on distance from the primary tumor (Wolff, E.M., et al., Unique DNA Methylation Patterns Distinguish Noninvasive and Invasive Urothelial Cancers and Establish an Epigenetic Field Defect in Premalignant Tissue, Cancer Research 70, 8169-8178). A similar widespread field defect was demonstrated during evaluation of Insulin-like Growth Factor 2 (IGF2) loss of imprinting in peripheral prostate tissues (Bhusari, S., Yang, B., Kueck, J., Huang, W. & Jarrard, D.F., Insulin-like growth factor-2 (IGF2) loss of imprinting marks a field defect within human prostates containing cancer, The Prostate, 2011 Mar 22). There has been recent interest in the treatment of PCa using focal ablative therapy (Mouraviev, V., et al., Prostate cancer laterality as a rationale of focal ablative therapy for the treatment of clinically localized prostate cancer, Cancer 110, 906-910 (2007)). The current findings suggest a field of susceptibility that might be utilized to help select patients who would be poor candidates for this approach.

In the current study, we focused on a high-resolution genome-wide analysis of methylation status rather than on specific gene promoter regions. The ENCODE18 human genome project includes gene-enriched areas thought to be biologically significant, a fact that potentially may generate a bias in our analyses. The majority of probes fell within CpG islands (Saxonov, S., Berg, P. & Brutlag, D.L., A genome-wide analysis of CpG dinucleotides in the human genome distinguishes two distinct classes of promoters, Proceedings of the National Academy of Sciences of the United States of America 103, 1412-1417 (2006); Fatemi, M., et al., Footprinting of mammalian promoters: use of a CpG DNA methyltransferase revealing nucleosome positions at a single molecule level, Nucleic Acids Research 33, e176), but none fell into defined gene promoter regions. Hypermethylation within promoters has been linked to decreased gene expression (JY, P., Promoter hypermethylation in prostate cancer, Cancer Control 17, 11; Cooper, C.S. & Foster, C.S., Concepts of epigenetics in prostate cancer development, Br J Cancer 100, 240-245 (2008)), but the function of CpG islands outside these regions remains uncertain. Given the potential for long-range epigenetic silencing, these changes may herald alterations in gene expression affecting distant regions (Clark, S.J., Action at a distance: Epigenetic silencing of large chromosomal regions in carcinogenesis, Human Molecular Genetics 16, R88-R95 (2007)), or, alternatively, reflect altered nuclear structure.

The current findings have several additional implications. PSA-based screening has been widely criticized for its failure to specifically identify lethal PCa (Adami, H.-O., The prostate cancer pseudo-epidemic, Acta Oncologica 49, 298-304). This study raises the possibility of using a tissue test, or potentially urine-based test, for the detection of disease (and specifically high-grade disease) based on abnormalities found in not only the tumor but in the associated TA tissues. This would be expected to demonstrate increased sensitivity by increasing the percentage of affected cells able to be detected. In addition, the assessment of alterations that occur in PCa have typically compared tumor to 'normal' tissues within the same prostate gland. The current study indicates that the histologically normal tissue from men who have PCa already contains methylation abnormalities, which may lead to an underestimation of epigenetic changes that exist in the associated cancers.

### Example 2

### Material and Methods

### Tissue Samples

Samples termed non-tumor associated (NTA, mean 63, age range 55-81 years old) were obtained from organ donation or cystoprostatectomy. The presence of any associated PCa was ruled out by extensive histological evaluation. Tumor-associated (TA, mean 61, age range 57-64 years old) prostate tissues were obtained from patients who underwent radical prostatectomy for PCa (Table 5). This study was approved by the institutional review boards at the University Pittsburgh and the University of Wisconsin-Madison. A separate validation group of 14 NTA (mean 60, age range 55-70 years old) and 12 TA (mean 58, age range 53-64 years old) samples were also assessed.

**Table 5**

| **Subject clinical and pathological characteristics** | | | | | |
|---|---|---|---|---|---|
| | **Methylation Array** | | **Pyrosequencing** | | |
| | **NTA** | **TA** | **NTA** | **TA** | **T, TAA, TAD** |
| **Number** | 5 | 4 | 14 | 11 | 26 |
| **Age (yr)** | 63 (55∼81) | 61 (57∼64) | 60 (55∼70) | 59 (51∼67) | 58 (44∼69) |
| **Tumor Volume (%)** | | 6.3 | | 5.1 | 27.1 |

| **Gleason grade** | | | | | |
|---|---|---|---|---|---|
| Intermediate | | 4 | | 6 | 16 |
| High | | | | | 10 |

| **Pathological stage** | | | | | |
|---|---|---|---|---|---|
| T2 | | | | 3 | |
| T2a | | | | 1 | 1 |
| T2b | | | | | 2 |
| T2c | | 3 | | 6 | 14 |
| T3a | | 1 | | 1 | 2 |
| T3b | | | | | 4 |
| PSA (ng/ml) | | 7.7 | | 5.9 | 6.9 |

| | | | | | |
|---|---|---|---|---|---|
| NTA: non-tumor-associated normal, TA: tumor-associate, T: tumor, TAA: tumor-associated adjacent, TAD: tumor-associated distant. Stages for three patients are unavailable. Intermediate: 3+3, 3+4; High:4+4, 4+5, 5+5. | | | | | |

To define the relationship of methylation to tumor foci, histological sections containing both cancer and normal regions were generated from 26 (mean 58, age range 44-69 years old) radical prostatectomy specimens under the direction of a genitourinary pathologist. Microdissection was performed to obtain tumor (T), normal tissue adjacent (2 mm) to tumor foci (TAA) and at a greater distance (10 mm, TAD) as previously described (Fig. 8) (Bhusari, S., Yang, B., Kueck, J., Huang, W. & Jarrard, D.F., Insulin-like growth factor-2 (IGF2) loss of imprinting marks a field defect within human prostates containing cancer, The Prostate, 2011 Mar 22). The clinical and pathological characteristics of the PCa study population are presented in Table 5. Of these patients, 16 had an intermediate grade cancer (Gleason score between 6 and 7; tumor volumes 5-70%) and 10 had high grade cancer (Gleason score 8-10; tumor volumes 25-80%). Prostate specimens were confirmed to have no tumor by both H&E staining in three dimensions and AMACR expression. For AMACR analysis, RNA was extracted using an RNeasy Mini Kit (Qiagen, CA), and 300 ng RNA was reverse transcribed with Ominscript® (Qiagen, CA). Quantitative real time PCR for total AMACR was performed using primer sequences as reported³³.

### DNA Methylation Microarrays

Genomic DNA was isolated using the DNeasy Blood & Tissue kit (Qiagen, CA). DNA used for microarray analysis was additionally incubated with RNaseA for 30 mins at 37°C to prevent any RNA contamination. Roche NimbleGen ENCODE HG18 DNA methylation arrays were utilized. These arrays contain 385,000 50-75mer oligonucleotides (probes) that cover biologically significant pilot regions of the human genome at 60-bp spacing.

Sample preparation for the microarray was performed following the manufacturer's protocol. Briefly, up to 6 micrograms of high-quality genomic DNA was digested with Msel (New England Biolabs, Ipswich, MA) to produce 200-1,000 bp fragments while keeping CpG islands intact, and was then heat denatured to single strand DNA fragments. Methylated DNA fragments were immunoprecipitated (IP) overnight at 4°C with 1µg of antibody against 5-methyl cytidine (Abcam, Cambridge, MA) and incubated with agarose beads for two hours. The DNA:antibody: bead mixture was digested with Proteinase K overnight at 55°C before purified with phenol-chloroform. Methylated immunoprecipited (MeDIP) DNA and flowthrough were validated with PCR primers specific for methylated and un-methylated regions as described by Weber et al (Weber, M., et al. Chromosome-wide and promoter-specific analyses identify sites of differential DNA methylation in normal and transformed human cells. Nat Genet 37, 853-862 (2005)). Enriched DNA was amplified with the WGA2 Kit (Promega, Madison, WI). The labeling of IP and input DNA, microarray hybridization and scanning were performed by NimbleGen (Reykjavik, Iceland) as described (Roche. NimbleGen Arrays User's Guide DNA Methylation Arrays Version 7.2, (2010). Data were extracted from scanned images using NimbleScan 2.4 extraction software (NimbleGen Systems, Inc.). The samples were assayed in duplicate.

### Sodium Bisulfite Modification and Quantitative Pyrosequencing

Sodium bisulfite modification of genomic DNA was carried out using the EpiTect Bisulfite Kit (Qiagen, CA) according to the manufacturer's protocol. Bisulfite modified DNA was then amplified using PCR with either the forward or reverse biotinylated primer in preparation for Pyrosequencing (Jörg Tost, El Abdalaoui, H., and Ivo Glynne Gut., Serial pyrosequencing for quantitative DNA methylation, BioTechniques, 40, 6 (2006)). The PCR and sequence primers for Pyrosequencing were designed using PyroMark Assay Design 2.0 (Qiagen), and positioned on or adjacent to the probe sites which showed significant (p < 0.01) methylation changes. The analyzed regions for specific loci are listed in Fig. 10, while primer sequences are listed in Fig. 12. The biotinylated PCR products were captured with Streptavidin sepharose beads, denatured to single strand and then annealed to the sequencing primer for the Pyrosequencing assay. Sssl methylase-treated bisulfite-converted DNA from HPEC (human prostate epithelial cell) and PPC1 cells were used as positive controls, and water substituted for DNA was used as a negative control. The methylation was quantified with the PyroMark™MD Pyrosequencing System (Qiagen, CA) within the linear range of the assay. All the samples were analyzed in at least two independent experiments, both in duplicate.

### Data Analysis

Scaled log₂-ratio GFF file and P-value GFF file were used for microarray analysis. These were extracted from scanned images provided by Nimblegen (NimbleGen Systems, Inc.). The scaled log₂-ratio data is the ratio of the test sample and input signals co-hybridized to the array. Scaling was performed by subtracting the bi-weight mean for all features of the array. From the scaled log₂-ratio data, a fixed-length window was placed around each consecutive probe and the one-sided Kolmogorov-Smirnov (KS) test was applied to determine whether the probes were drawn from a significantly more positive distribution of intensity log-ratios than those in the rest of array. The resulting score for each probe is the -log₁₀ p-value. The probe IDs were first chosen based on a p-value -log₁₀ [p] that ranged from 2 to 10 resulting in around 1,000 probes on each chromosome and 18,101 probes in total. After statistical analysis comparing the log₂-ratios between the NTA and TA groups, significant methylation differences between groups were determined using t-test (P < 0.05). Significantly changed probes were clustered by Java MultiExperiment View (MEV 4.6.2) with unsupervised Hierarchical Clustering (Saeed AI, B.N., Braisted JC, Liang W, Sharov V, Howe EA, et al., TM4 microarray software suite, Methods in Enzymology 411, 60 (2006)).

For quantitative Pyrosequencing, the methylation at each CpG site was expressed as a percentage. A t-test was used to test for differences between groups, P < 0.05 was considered statistically significant. The Spearman test was used to determine correlations, with significance set at P < 0.05; r represents the measure of the relationship between two variables, and varies from -1 to +1.

### Example 3

### CpG Islands

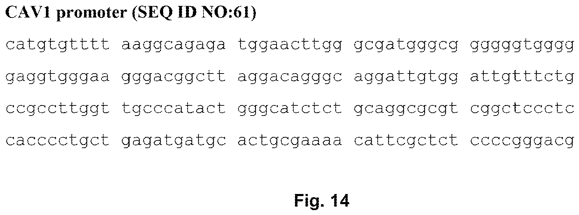

Based on the teachings of Examples 1 and 2, one can also check the CpG islands that are located in the promoter regions of the genes showing significant methylation changes correlating with PCa, preferably the region within about 5kb upstream of the transcription start site (TSS), because the methylation of these CpG islands will change the gene expressions and affect gene functions. The inventors' primary research (data not shown) showed that one may wish to start with genes CAV1, EVX1, MCF2L and WNT2. The expanded regions of each of the six genes for preferred screening of methylation changes are detailed in Figs. 14-19.

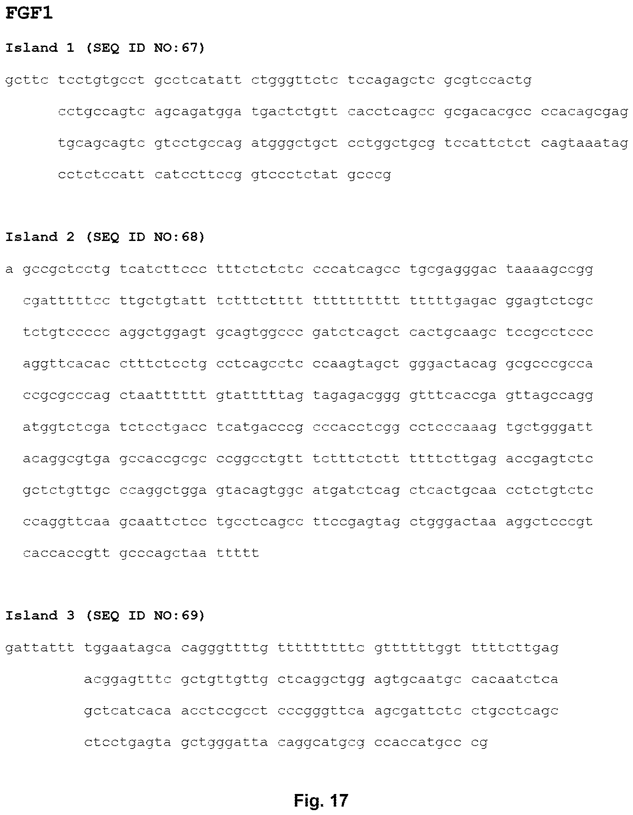

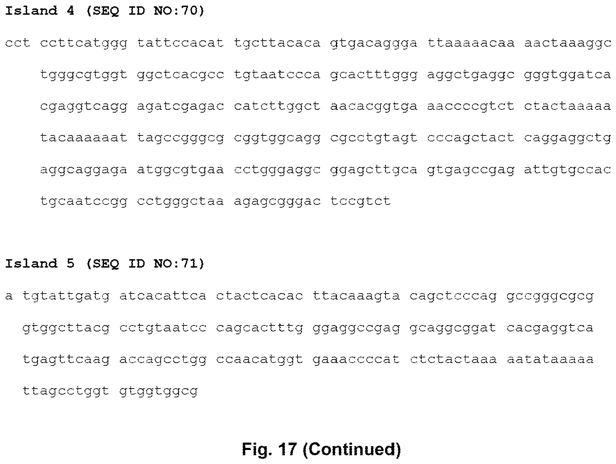

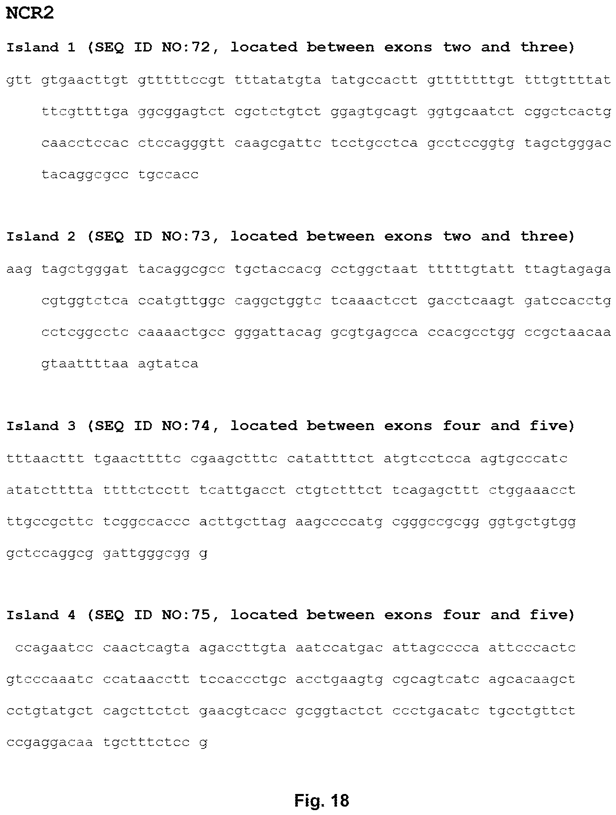

FGF1 and NCR2 do not have CpG islands within the promoter regions. For FGF1, the expanded regions for preferred screening of methylation changes would be 300bps upstream and 1kb downstream of the target region reported in Example 1, as well as about 5Kb upstream of the translation start site ATG (detailed in Fig. 17). For NCR2 the expanded regions for preferred screening of methylation changes would be the region between exon two and three and the two CpG islands between exon four and five (detailed in Fig. 18).

### Example 4

### Development Of A DNA Methylation Urine-Based Screen For Lethal PCa

As disclosed in Example 1, specific loci associated with field defect appear to be preferentially altered in lethal, high grade PCa, which is responsible for the majority of PCa deaths. Establishing the role epigenetic changes play in the development of lethal PCa can lead to better diagnosis and treatment of high grade PCa. We envision that epigenetic field defect characterized by changes in DNA methylation in histologically normal appearing cells within the prostate can be utilized to identify patients with lethal disease.

### Introduction

In 2010, PCa was the most commonly diagnosed cancer in Wisconsin men (Fu VX, Dobosy JR, Desotelle JA, Almassi N, Ewald JA, Srinivasan R, Berres M, Svaren J, Weindruch R, Jarrard DF., Aging and cancer-related loss of insulin-like growth factor 2 imprinting in the mouse and human prostate, Cancer Res. 2008 Aug 15;68(16):6797-802), and is the second most common cause of cancer death (after lung cancer), with over 600 men succumbing to the disease (Jemal A, Siegel R, Xu J, Ward E., Cancer statistics, 2010. 1. CA Cancer J.Clin. 2010 Sep;60(5):277-300). Over 70% of PCa deaths occur in men diagnosed with high grade (Gleason Score 8-10) disease or high volume intermediate grade disease (Gleason Score 6-7), making the detection of these variants at an earlier time point critical (Stephenson A.J., Kattan M.W., Eastham J.A., Bianco F.J., Jr., Yossepowitch O., Vickers A.J., Klein E.A., Wood D.P., Scardino P.T., Prostate cancer-specific mortality after radical prostatectomy for patients treated in the prostate-specific antigen era, J.Clin.Oncol. 2009 Sep 10;27(26):4300-5). Low volume (<10%) intermediate and lower grade cancers have a much more indolent natural history. Several striking features of PCa include its multifocality and marked increase in incidence with aging. These characteristics suggest a 'field defect' may be an important component in the etiology of PCa. To date, cancer diagnosis has focused on the finding of cancer cells, typically by biopsy, yet the presence of alterations associated with histologically normal prostate tissue is as yet an untapped resource in both the diagnosis and understanding of the etiology of this disease.

Over 600,000 diagnostic prostate biopsies are performed annually in the United States. The false negative rate is as high as 34%, and roughly 20-35% of patients sent for repeat biopsy are ultimately diagnosed with cancer (Djavan B, Zlotta A, Remzi M, Ghawidel K, Basharkhah A, Schulman CC, Marberger M. Optimal predictors of prostate cancer on repeat prostate biopsy: A prospective study of 1,051 men, J.Urol. 2000 Apr.;163(4):1144-8). Prostate biopsy is associated with risk of bleeding, urinary distress and hospitalization for infection that increases with each subsequent biopsy. Alternatively, patients whose biopsies are initially negative with an elevated PSA represent a serious clinical dilemma, and are at risk for additional evaluation costs and procedures, including saturation biopsy that is performed in the operating room under anesthesia. Men in this situation experience significant anxiety as well (Katz DA, Jarrard DF, McHorney CA, Hillis SL, Wiebe DA, Fryback DG., Health perceptions in patients who undergo screening and workup for prostate cancer, Urology 2007 Feb;69(2):215-20). The development of a non-invasive test to augment PSA screening would be of enormous benefit to society.

Currently utilized screening tests (serum prostate specific antigen (PSA) and digital rectal exam have only a modest predictive value (Strope SA, Andriole GL, Prostate cancer screening: Current status and future perspectives, Nat.Rev.Urol. 2010 Sep;7(9):487-93). PSA isoforms add little specificity. Body fluids including semen and urine may contain molecular information regarding the presence of PCa. PCa and prostate epithelial cells are shed into biologic fluids, particularly when the prostate is subjected to physical manipulation, thus creating the potential for their noninvasive detection in either urine or expressed prostatic fluid. Attempts at detecting PC cells in urine by traditional cytology are thwarted by unacceptably low sensitivities, although specificities were consistently high (Fujita K., Pavlovich C.P., Netto G.J., Konishi Y., Isaacs W.B., Ali S., DeMarco A., Meeker A.K., Specific detection of prostate cancer cells in urine by multiplex immunofluorescence cytology, Hum.Pathol. 2009 Jul;40(7):924-33). This is due primarily to low numbers of PC cells present in urine cytology preparations. Analyzing cells shed from the abnormal prostate bypasses this important hurdle and represents the first effort of its kind in prostate and many other cancers.

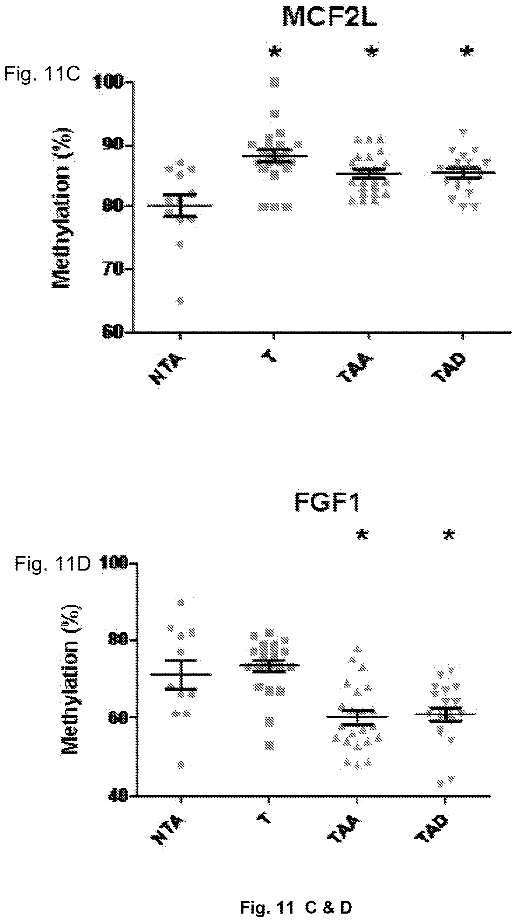

To date, one of the few field defect alterations found in both non-cancerous peripheral prostate tissue and in associated prostate tumors is our finding of a loss in the typical imprint of the IGF2 gene (Fu V.X., Dobosy J.R., Desotelle J.A., Almassi N., Ewald J.A., Srinivasan R., Berres M., Svaren J., Weindruch R., Jarrard D.F., Aging and cancer-related loss of insulin-like growth factor 2 imprinting in the mouse and human prostate, Cancer Res. 2008 Aug 15;68(16):6797-802). We have demonstrated that this is not a peritumor phenomenon (i.e. adjacent response to the cancer), but is widely prevalent even in distant areas within the peripheral prostate (Bhusari S., Yang B., Kueck J., Huang W., Jarrard D.F., Insulin-like growth factor-2 (IGF2) loss of imprinting marks a field defect within human prostates containing cancer, Prostate 2011 Mar 22). Our lab has expanded these studies to other epigenetic phenomenon and recently using a series of Nimblegen™ ENCODE18 Methylation Arrays, which survey the whole human genome, have identified 87 loci (out of 385,000 loci surveyed) that exhibit altered methylation (p<0.01) in the peripheral prostate tissue of men who have the disease when compared to those that do not (Fig. 9D). Interestingly these methylation defects are found both in gene and relatively gene-free areas of the genome. To date, we have screened 16 of these loci and validated 6 (CAV1, EVX1, MCF2L, FGF1, WNT2 and NCR2) using quantitative bisulfite Pyrosequencing in an additional cohort of 40 patients (Fig. 11). Notably, we found that methylation at the WNT2 and NCR2 were associated with the field defect in high grade, but not intermediate grade, cancers (Fig. 11E-F). This striking finding suggests these high grade cancers may have a molecular fingerprint present in the adjacent normal tissues that could assist in the earlier diagnosis of the disease. Finally, analyses of associations between tumor volume, PSA, and the extent of methylation demonstrated a significant association between FGF1 and increased tumor volume (P = 0.036, r = 0.4616) (see Example 1). In addition to histological confirmation of the absence of cancer in these prostate tissues, we also performed AMACR expression analysis, a specific marker for the presence of PCa (Ananthanarayanan V., Deaton R.J., Yang X.J., Pins M.R, Gann P.H., Alpha-methylacyl-CoA racemase (AMACR) expression in normal prostatic glands and high-grade prostatic intraepithelial neoplasia (HGPIN): association with diagnosis of prostate cancer, Prostate 2005 Jun 1;63(4):341-6), to rule out contamination with cancer cells (data not shown). In sum, these data demonstrate that particular methylation changes occur at specific loci in tumor associated tissues and that several of these markers are altered preferentially in high grade cancers.

### Significance

By defining these epigenetic changes one can leverage this information to improve diagnosis and cure of high grade PCa. This analysis has the potential to provide an assay that will decrease the morbidity associated with PCa diagnosis and improve prognostication. This panel of markers can be used on non-cancer prostate biopsy tissue to validate negative findings and decrease in the near term the number and frequency of biopsies being performed in men with elevated PSAs. In addition, we envision the application of these markers to develop a non-invasive urine test that can be used as an adjunct to further identify men with a higher risk lethal PCa. The approaches to achieve these goals are described in detail below.

### Confirm that methylation alterations associated with a field defect in high grade/high volume PCa can be detected in the urine (Prophetic Example)

Prostate cells are shed into the urine. Previous small studies have focused on cancer-specific methylation alterations in the urine (Fujita K., Pavlovich C.P., Netto G.J., Konishi Y., Isaacs W.B., Ali S., De Marco A., Meeker A.K., Specific detection of prostate cancer cells in urine by multiplex immunofluorescence cytology, Hum.Pathol. 2009 Jul;40(7):924-33; Rogers C.G., Gonzalgo M.L., Yan G., Bastian P.J., Chan D.Y., Nelson W.G., Pavlovich C.P., High concordance of gene methylation in post-digital rectal examination and post-biopsy urine samples for prostate cancer detection, J.Urol. 2006 Nov;176(5):2280-4) and have demonstrated feasibility, but lower sensitivity because of the presence of rare cancer cells. In contrast, normal prostate epithelial cells are found within the urine at a much higher rate (Fujita K., Pavlovich C.P., Netto G.J., Konishi Y., Isaacs W.B., Ali S., De Marco A., Meeker A.K., Specific detection of prostate cancer cells in urine by multiplex immunofluorescence cytology, Hum.Pathol. 2009 Jul;40(7):924-33). We seek to evaluate methylation changes found in normal cells associated with prostate cancer to determine if these changes predict the presence of cancer within this biofluid. Notably, our markers are also abnormal in cancer cells.

We will take validated tissue markers (six markers disclosed in Example 1 and others validated from the above described experiments in this Example) and apply them to urine specimens from men undergoing prostate biopsy throughout Wisconsin. We will confirm that methylation differences can be detected in the urine from men with cancer versus those without.

We envision that prospective urine samples from 250 men with high PSA values undergoing prostate biopsy will be obtained after an 'attentive' digital rectal examination. Of these samples 100 will be obtained through the Wisconsin Network for Health Research (WNHR). A further control group of 50 age-matched controls seen in the urology clinic with normal PSA values will be consented, obtained and tested. Briefly, after prostate examination, 20ml of the initial stream will be collected, mixed with EDTA and stored on ice as described (Rogers C.G., Gonzalgo M.L., Yan G., Bastian P.J., Chan D.Y., Nelson W.G., Pavlovich C.P., High concordance of gene methylation in post-digital rectal examination and post-biopsy urine samples for prostate cancer detection, J.Urol. 2006 Nov;176(5):2280-4).

Genomic DNA will be extracted from the pellet using a column as above. DNA will then be sodium bisulfite treated and quantitative Pyrosequencing performed using our panel of loci CAV1, EVX1, MCF2L, FGF1 and NCR2, as well as additional markers validated from the above described experiments in this Example. Methylation of individual loci will be compared between the TA and NTA groups using two-tailed student's t-tests conducted at a significance level of 0.026 (a rough false discovery rate). Additional analyses will be performed using logistic regression to determine if multiple loci, total PSA, free PSA, PSA density, or age improves the ability to predict which individuals belong to the TA group. Assuming that 150 of the 300 subjects belong to the TA group and the other 150 belong to the NTA group, we will have at least 80% power for detecting as significant a 0.3557 standard deviation shift in the mean methylation value between groups. Further subgroup analyses will be performed based on tumor volume, age, pathologic stage, and cancer grade.

In conjunction with the above approaches, we will seek to develop alternate technologies to quantitate methylation to permit widespread application. The original Nimblegen methylation arrays allows detection of methylation at specific sites, but not at basepair resolution. However, complete analysis of the prognostic potential of these sites will require a thorough analysis of the entire locus to identify specific nucleotides where methylation is predictive of disease course. Although the pyrosequencing approach is an established technique within our laboratory, one of its limitations is that it can only scan a limited number of methylation sites encompassing 100-300 bp within a single run and it is time consuming and expensive.

We will confirm alternate technologies which improve assay sensitivity and commercial applicability by: i) developing a methylation-sensitive qPCR multiplex approach based on amplification of multiple specific methylated loci (Campan M., Weisenberger D.J., Trinh B., Laird P.W. MethyLight. Methods Mol.Biol. 2009;507:325-37), and ii) implementing direct sequencing of samples by utilizing next generation sequencing technology (available from the UW Biotech Center) to digitally detect methylation sites at basepair resolution. We will rely on methylation-specific priming combined with both methylation and unmethylation-specific fluorescent probes. This assay is faster with an accompanying ability to sensitively detect very low frequencies of hypermethylated alleles (Campan M., Weisenberger D.J., Trinh B, Laird PW. MethyLight. Methods Mol.Biol. 2009;507:325-37). Direct sequencing utilizes established sequence capture techniques (for 25-30 loci) and then methylation analyses as described (Gu H., Smith Z.D., Bock C., Boyle P., Gnirke A., Meissner A., Preparation of reduced representation bisulfite sequencing libraries for genome-scale DNA methylation profiling, Nat.Protoc. 2011 Apr;6(4):468-81). Briefly, the Agilent Sureselect™ system will be used to capture approximately 50 kb nucleotides surrounding each of these loci (approximately 0.1% of entire genome) for at least 100 of the samples. The enriched samples can be barcoded and sequenced in a high-throughput fashion using the Illumina HiSeq™ instrument (or a similar alternate machine) at the UW Biotechnology Center (80 million reads/lane) to identify specific sites of methylation by comparing sequences with bisulfite-converted material, thus providing a digital readout on the percentage of methylation at a specific site in a given sample.

We anticipate being able to detect methylation differences at one or multiple loci in men that have cancer and specifically high grade cancer. By increasing the pool of markers validated in tissues, we will decrease the likelihood that significant markers will not be detected in urine. Given the markers in TA prostate tissues identified so far are also abnormal in the cancer themselves, we anticipate the sensitivity of this approach will be much higher than approaches with markers specifically altered in cancer (Fujita K., Pavlovich C.P., Netto G.J., Konishi Y., Isaacs W.B., Ali S., De Marco A., Meeker A.K. Specific detection of prostate cancer cells in urine by multiplex immunofluorescence cytology. Hum.Pathol. 2009 Jul;40(7):924-33). Statistical analyses for the methylated loci will likely be improved by the use of PSA, family history, digital rectal exam in statistical analyses.

We perform roughly 500 prostate biopsies a year at UW providing a larger pool of urine samples if necessary. Obtaining urine samples from the Wisconsin Network for Health Research (WNHR) will validate our finding to patients throughout Wisconsin. Roughly 10ug of DNA can be extracted from 20ml of urine using this approach (Rogers C.G., Gonzalgo M.L., Yan G., Bastian P.J., Chan D.Y., Nelson W.G., Pavlovich C.P., High concordance of gene methylation in post-digital rectal examination and post-biopsy urine samples for prostate cancer detection, J.Urol. 2006 Nov;176(5):2280-4). The presence of competing cells of other etiology (including bladder, kidney and WBC) may have altered methylation changes. If this is encountered we will seek to enrich for the prostate cell population by utilizing antibodies to anti-NKX3.1 as described (Fujita K., Pavlovich C.P., Netto G.J., Konishi Y., Isaacs W.B., Ali S., De Marco A., Meeker A.K. Specific detection of prostate cancer cells in urine by multiplex immunofluorescence cytology, Hum.Pathol. 2009 Jul;40(7):924-33). Given the cancer association of the markers identified, it would be unlikely other cell types will be altered in normal tissues from other sources.

### Example 5

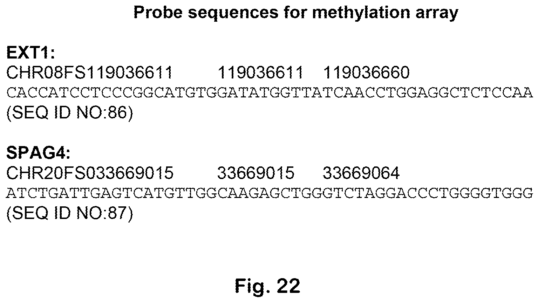

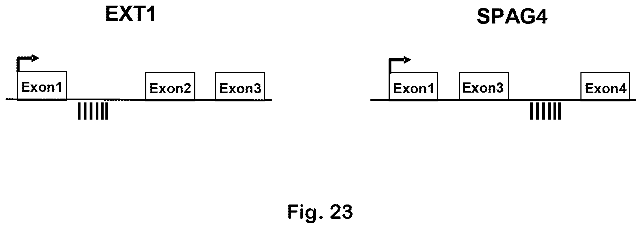

In an experiment analogous to Example 1, a subset of two genes was chosen for further evaluation, based on genomic location, putative biological function, extent of methylation and primer success in a separate validation using a set of 24 TA and NTA prostate specimens. Quantitative Pyrosequencing was employed to allow a more accurate evaluation of the extent of DNA methylation. Internal controls for the adequacy of bisulfite conversion were performed. Two loci, which were associated with the genes EXT1 and SPAG4 showed significant methylation changes (P < 0.05). The locus associated with SPAG4 was hypermethylated and the locus associated with EXT1 was hypomethylated. The location of the probes and CG's assessed by Quantitative Pyrosequencing are shown in Figs. 23 and 25. The two loci in pyrosequencing are close or overlap the methylation array regions but sequences (Fig. 22) are different. The sequences listed in Fig 20-21 have covered both array region (Fig 22) and pyrosequencing regions. These data demonstrate that TA tissues have a methylation profile distinct from men without cancer (NTA) and that these changes alter specific regions of the genome.

### Identification of a widespread methylation field defect in the peripheral prostate.

Preferential alteration in tissues adjacent to PCa tumor foci, i.e., field defect, suggests a peritumoral response. To evaluate whether tissues adjacent to PCa tumor foci are preferentially altered, the extent of field defect was assessed in 26 additional histologically normal tissues by looking at the methylation status of these two differentially methylated markers. The inventors micro-dissected normal tissues adjacent (TAA, 2 mm) and distant (TAD, >10 mm) from the main tumor focus for each of the specimens (Fig. 8). Histological 3-dimensional H&E staining and AMACR expression determined by qPCR were applied to rule out any contamination by tumor cells or the presence of high grade prostatic intraepithelial neoplasia (HGPIN), a putative cancer precursor (Ayala, A.G. & Ro, J.Y. Prostatic Intraepithelial Neoplasia: Recent Advances. Archives of Pathology & Laboratory Medicine 131, 1257-1266 (2007)). Increased AMACR expression was found in two NTA and three TA tissues that were subsequently excluded from further analysis (Fig 13).

When compared to NTA tissues, hypermethylation of probes associated with SPAG4 and hypomethylation of EXT1 demonstrated significant changes in both TAA, as well as TAD tissues (Fig. 24 and Table 6). Notably, there was no difference in the extent of methylation seen at different distances from the tumor when TAA and TAD tissue sets were compared. Significant methylation changes were also seen in tumor samples when compared to NTA tissues for EXT1 and SPAG4, revealing a persistence of these changes in the associated cancer. These data indicate that the epigenetic field defect in the prostate is widespread and not solely localized to the immediate peritumor environment.

**Table 6 - Methylation Percentage Of All Analyzed CpGs For Each Gene**

| | **EXT1** | | | **SPAG4** | | |
|---|---|---|---|---|---|---|
| | **NTA** | **TAA** | **TAD** | **NTA** | **TAA** | **TAD** |
| **CG1** | **39.4** | **34.7*** | **34.2*** | **13.5** | **21.4*** | **25.2*** |
| **CG2** | **28.3** | **24.1*** | **24.5*** | **15.9** | **25.4*** | **27.3*** |
| **CG3** | **38.2** | **35.1*** | **35.0*** | **16.1** | **18.7*** | **18.1** |
| **CG4** | **27.2** | **24.3*** | **24.0*** | **11.6** | **15.9*** | **15.6*** |
| **CG5** | **14.8** | **12.8** | **14.0** | **9.0** | **11.5*** | **10.8** |
| **CG6** | **32.5** | **36.3** | **38.5** | | | |

| | | | | | | |
|---|---|---|---|---|---|---|
| *P<0.05 | | | | | | |

### Example 6

### CpG Islands

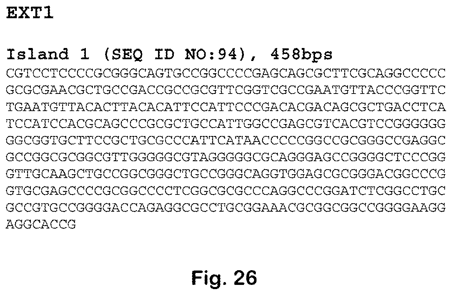

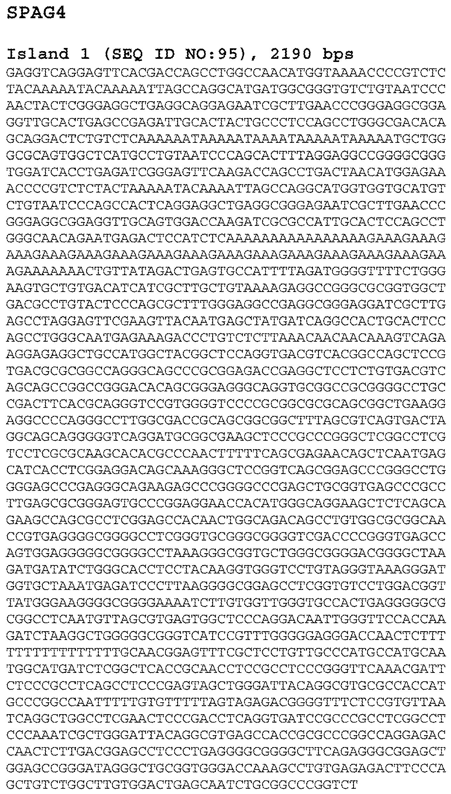

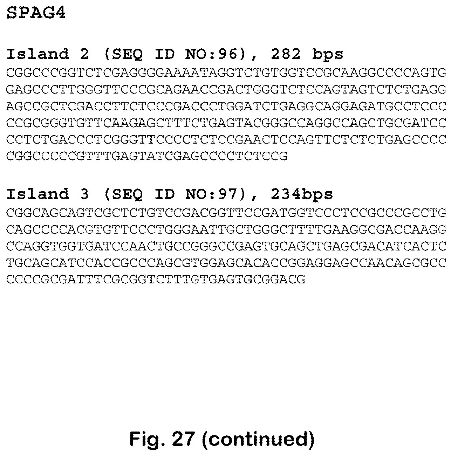

Based on the teachings of Examples 1, 2 and 5, one can also check the CpG islands that are located in the promoter regions of the genes showing significant methylation changes correlating with PCa, preferably the region within about 5kb upstream of the transcription start site (TSS), because the methylation of these CpG islands will change the gene expressions and affect gene functions. The inventors' primary research (data not shown) showed that one may wish to examine genes EXT1 and SPAG4. The expanded regions of each of these two genes for preferred screening of methylation changes are detailed in Figs. 26-27.

Both EXT1 and SPAG4 have CpG islands within the promoter regions. For EXT1, the expanded regions for preferred screening of methylation changes would be from 373bps upstream to 84 downstream of transcription start site (TSS) Fig. 26 (SEQ ID NO:94). For SPAG4 the expanded regions for preferred screening of methylation changes would be from 1100bps upstream of TSS through the first exon (SEQ ID NO:95), 1180bps down stream of TSS (intron 1 and exon2, SEQ ID NO:96) and 3640bps down stream of TSS (intron 9 and exon10, SEQ ID NO:97).

### Example 7

### DNA Methylation Urine-Based Screen For PCa

A widespread epigenetic field defect can be used to detect prostate cancer in patients with histologically negative biopsies (Truong et al., "Using the Epigenetic Field Defect to Detect Prostate Cancer in Biopsy Negative Patients" (2012) J Urol*, in press*). Prostate biopsies are performed on the patients who have elevated PSA levels. Prostatic massage will be given to each patient to increase the amount of prostate cells voided in the urine, and then voided urine will be collected from them. Those patients classified as having adenocarcinoma will be used in the positive biopsy samples, and the patients with this current biopsy negative and all previous negative biopsy will be used in the negative biopsy samples. The urine is centrifuged for 15 minutes at 1200 rpm at 4°C, the excess supernatant is removed and pellet at -80°C immediately.

Genomic DNA from urine and biopsy tissue is extracted using Qiagen DNeasy Blood and Tissue Kit, Bench Protocol: Animal Tissues (Qiagen). The DNA is then treated with sodium bisulfite using the Qiagen EpiTect Bisulfite Handbook protocol (Qiagen, Valencia, CA) to modify the DNA to turn all the unmethylated cytosine to uracil. The bisulfite modified DNA is amplified by polymerase chain reaction (PCR) using gene specific primers, with either the forward or reverse primer biotinylated. The genes amplified include CAV1, EVX1, WNT2, MCF2L, NCR2, FGF1, EXT1 and SPAG4. Five microliter of the PCR products will be applied for Pyrosequencing to ascertain the actual percent methylation within the gene. The assay is run in a PyroMark™MD Pyrosequencing System (Qiagen). All samples are analyzed with two independent trials and t-test will be used to test for differences in methylation between the positive and negative biopsy urine samples with p < 0.05 considered statistically significant.

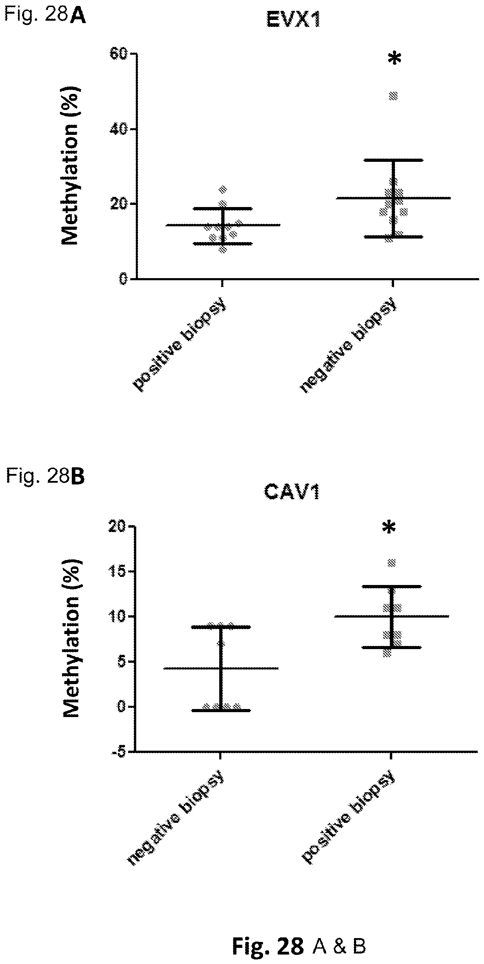

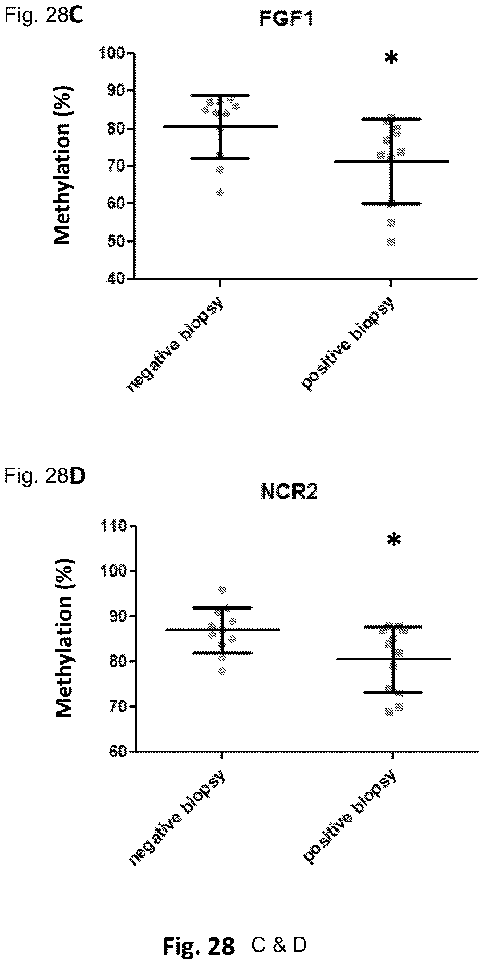

Fig. 28 shows methylation of the genes in urine from the patients who have either positive or negative biopsies for prostate cancer. We have tested the methylation for the six markers EVX1, CAV1, FGF1, MCF2L, WNT2 and NCR2. EVX1, CAV1, FGF1 and NCR2 showed significant methylation difference between the biopsy positive and negative groups, t-test *P<0.05.

## Claims

1. A method of diagnosing prostate cancer in a human subject comprising quantitating methylation in genomic DNA obtained from the human subject in at least one target region selected from the group consisting of SEQ ID NO: 18 and SEQ ID NO:39, wherein significant methylation changes indicate the presence of prostate cancer or a prostate cancer field defect, wherein the change is relative to tissue from a second human subject who does not have prostate cancer.

2. The method of claim 1, wherein the genomic DNA is obtained from prostate tissue.

3. The method of claim 1, wherein the genomic DNA is obtained from body fluid selected from the group consisting of plasma, urine and semen.

4. The method of claim 1, wherein the quantitation comprises analyzing whether the target region of SEQ ID NO: 39 is hypermethylated and the target region of SEQ ID NO: 18 is hypomethylated as a positive correlation to prostate cancer field defect.

5. The method of claim 4, wherein the target regions are amplified using at least one set of primers in SEQ ID NOs: 88-93.

6. A method of amplifying at least one of target DNA sequence to diagnose prostate cancer in a subject comprising the steps of
(a) providing a reaction mixture comprising a double-stranded bisulfite modified target DNA of a subject and (i) at least one pair of primers selected from the group designed to amplify at least one gene selected from the group consisting of SEQ ID NO: 18 and SEQ ID NO: 39, wherein the primer pair comprises a first and a second primer that are complementary to the target DNA sequence, (ii) a polymerase and (iii) a plurality of free nucleotides comprising adenine, thymine, cytosine and guanine;
(b) heating the reaction mixture to a first predetermined temperature for a first predetermined time to separate the strands of the target DNA from each other;
(c) cooling the reaction mixture to a second predetermined temperature for a second predetermined time under conditions to allow the first and second primers to hybridize with their complementary sequences on the target DNA and to allow the polymerase to extend the primers; and
(d) repeating steps (b) and (c) at least 10 times, wherein increased methylation, relative to tissue from a second human subject who does not have prostate cancer, indicates the presence of prostate cancer or a prostate cancer field defect.

7. Use of a kit for prostate cancer diagnosis or prostate cancer field defect detection, the kit comprising at least a set of primers, wherein the primer set consists of a forward primer and a reverse primer designed to amplify a target region selected from the group consisting of SEQ ID NOs: 18 and 39, and components essential for DNA amplification.

8. Use of the kit of claim 7, wherein the primer set consists of a forward primer selected from the group consisting of SEQ ID No: 88 and 91, and a reverse primer selected from the group consisting of SEQ ID No: 89 and 92.

9. Use of the kit of claim 87, wherein the components essential for DNA amplification comprise polymerase, dNTPs, and a magnesium salt which can release Mg²⁺.

10. Use of the kit of claim 7, wherein the kit further comprises FAM or Hex fluorophore-labeled methylation and unmethylation-specific probes and is suitable for a closed tube assay for MS-QPCR.

11. Use of the kit of claim 7, wherein the kit further comprises sequencing primers and is suitable for bisulfite pyrosequencing-based assay.

12. Use of the kit of claim 7, wherein the sequencing primers are selected from the group consisting of SEQ ID NOs: 90 and 93.

13. Use of the kit of claim 7, further comprising Streptavidin sepharose beads, enzyme mixture, substrate mixture and dinucleotides.

14. Use of the kit of claim 7, wherein the kit comprises sets of primers selected from the group consisting of two sets, three sets, four sets, five sets, six sets, seven sets and eight sets.

## Patentansprüche

1. Verfahren zum Diagnostizieren von Prostatakrebs bei einem menschlichen Subjekt, umfassend ein Quantifizieren von Methylierung in genomischer DNA, die von dem menschlichen Subjekt erlangt wird, in mindestens einer Zielregion, ausgewählt aus der Gruppe bestehend aus SEQ ID NO: 18 und SEQ ID NO: 39, wobei signifikante Methylierungsveränderungen das Vorhandensein von Prostatakrebs oder einen Prostatakrebs-Felddefekt angeben, wobei die Veränderung in Bezug auf Gewebe von einem zweiten menschlichen Subjekt ist, das keinen Prostatakrebs hat.

2. Verfahren nach Anspruch 1, wobei die genomische DNA aus Prostatagewebe erlangt wird.

3. Verfahren nach Anspruch 1, wobei die genomische DNA aus einer Körperflüssigkeit erlangt wird, ausgewählt aus der Gruppe bestehend aus Plasma, Urin und Sperma.

4. Verfahren nach Anspruch 1, wobei die Quantifizierung ein Analysieren umfasst, ob die Zielregion von SEQ ID NO: 39 hypermethyliert ist und die Zielregion von SEQ ID NO: 18 hypomethyliert ist, als eine positive Korrelation zu Prostatakrebs-Felddefekt.

5. Verfahren nach Anspruch 4, wobei die Zielregionen unter Verwendung mindestens eines Satzes von Primern in SEQ ID NO: 88-93 amplifiziert werden.

6. Verfahren zum Amplifizieren von mindestens einer Ziel-DNA-Sequenz, um Prostatakrebs bei einem Subjekt zu diagnostizieren, umfassend die folgenden Schritte:
(a) Bereitstellen eines Reaktionsgemischs, umfassend eine doppelsträngige Bisulfitmodifizierte Ziel-DNA eines Subjekts und (i) zumindest ein Primerpaar, ausgewählt aus der Gruppe, die konstruiert ist, um zumindest ein Gen, ausgewählt aus der Gruppe bestehend aus SEQ ID NO: 18 und SEQ ID NO: 39, zu amplifizieren, wobei das Primerpaar einen ersten und einen zweiten Primer umfasst, die komplementär zu der Ziel-DNA-Sequenz sind, (ii) eine Polymerase und (iii) eine Vielzahl von freien Nukleotiden, umfassend Adenin, Thymin, Cytosin und Guanin;
(b) Erwärmen des Reaktionsgemischs auf eine erste vorbestimmte Temperatur über eine erste vorbestimmte Zeit, um die Stränge der Ziel-DNA voneinander zu trennen;
(c) Abkühlen des Reaktionsgemischs auf eine zweite vorbestimmte Temperatur über eine zweite vorbestimmte Zeit unter Bedingungen, die es dem ersten und dem zweiten Primer erlauben, mit ihren komplementären Sequenzen an der Ziel-DNA zu hybridisieren und der Polymerase erlauben, die Primer zu verlängern; und
(d) Wiederholen der Schritte (b) und (c) mindestens 10 Mal, wobei eine erhöhte Methylierung in Bezug auf Gewebe von einem zweiten menschlichen Subjekt, das keinen Prostatakrebs hat, das Vorhandensein von Prostatakrebs oder einen Prostatakrebs-Felddefekt angibt.

7. Verwendung eines Kits zur Diagnose von Prostatakrebs oder zum Detektion eines Prostatakrebs-Felddefekts, das Kit umfassend zumindest einen Satz von Primern, wobei der Primersatz aus einem Vorwärtsprimer und einem Rückwärtsprimer, die konstruiert sind, um eine Zielregion, ausgewählt aus der Gruppe bestehend aus SEQ ID NO: 18 und 39, zu amplifizieren, und Komponenten, die für eine DNA-Amplifikation wesentlich sind, besteht.

8. Verwendung des Kits nach Anspruch 7, wobei der Primersatz aus einem Vorwärtsprimer, ausgewählt aus der Gruppe bestehend aus SEQ ID NO: 88 und 91, und einem Rückwärtsprimer, ausgewählt aus der Gruppe bestehend aus SEQ ID NO: 89 und 92, besteht.

9. Verwendung des Kits nach Anspruch 7, wobei die Komponenten, die für eine DNA-Amplifikation wesentlich sind, Polymerase, dNTPs und ein Magnesiumsalz, das Mg²⁺ freisetzen kann, umfassen.

10. Verwendung des Kits nach Anspruch 7, wobei das Kit ferner mit Fluorphor markierte Methylierungs- und Unmethylierungs-spezifische FAM- oder Hex-Sonden umfasst und für einen geschlossenen Röhrchentest für MS-QPCR geeignet ist.

11. Verwendung des Kits nach Anspruch 7, wobei das Kit ferner Sequenzierungsprimer umfasst und für einen auf Bisulfit-Pyrosequenzierung-basierenden Test geeignet ist.

12. Verwendung des Kits nach Anspruch 7, wobei die Sequenzierungsprimer ausgewählt sind aus der Gruppe bestehend aus SEQ ID NOs: 90 und 93.

13. Verwendung des Kits nach Anspruch 7, ferner umfassend Streptavidin-Sepharose-Kügelchen, Enzymgemisch, Substratgemisch und Dinukleotide.

14. Verwendung des Kits nach Anspruch 7, wobei das Kit Primersätze umfasst, die ausgewählt sind aus der Gruppe bestehend aus zwei Sätzen, drei Sätzen, vier Sätzen, fünf Sätzen, sechs Sätzen, sieben Sätzen und acht Sätzen.

## Revendications

1. Procédé de diagnostic du cancer de la prostate chez un sujet humain comprenant la quantification de la méthylation dans l'ADN génomique obtenu à partir du sujet humain dans au moins une région cible sélectionnée dans le groupe constitué des SEQ ID NO: 18 et SEQ ID NO: 39, dans laquelle une méthylation significative des changements indiquent la présence d'un cancer de la prostate ou d'un défaut de champ du cancer de la prostate, dans lequel le changement est relatif au tissu d'un deuxième sujet humain qui n'a pas de cancer de la prostate.

2. Procédé selon la revendication 1, dans lequel l'ADN génomique est obtenu à partir de tissu prostatique.

3. Procédé selon la revendication 1, dans lequel l'ADN génomique est obtenu à partir d'un fluide corporel choisi dans le groupe constitué par le plasma, l'urine et le sperme.

4. Procédé selon la revendication 1, dans lequel la quantification comprend l'analyse si la région cible de SEQ ID NO: 39 est hyperméthylée et la région cible de SEQ ID NO: 18 est hypométhylée en tant que corrélation positive avec un défaut de champ du cancer de la prostate.

5. Procédé selon la revendication 4, dans lequel les régions cibles sont amplifiées en utilisant au moins un ensemble d'amorces dans les SEQ ID NO: 88-93.

6. Procédé d'amplification d'au moins une séquence d'ADN cible pour diagnostiquer un cancer de la prostate chez un sujet comprenant les étapes de
(a) fournir un mélange réactionnel comprenant un ADN cible modifié par bisulfate double brin d'un sujet et (i) au moins un paire d'amorces sélectionnées dans le groupe conçu pour amplifier au moins un gène sélectionné dans le groupe constitué des SEQ ID NO: 18 et SEQ ID NO: 39, dans lequel la paire d'amorces comprend une première et une deuxième amorce qui sont complémentaires à l'ADN cible séquence, (ii) une polymérase et (iii) une pluralité de nucléotides libres comprenant l'adénine, la thymine, la cytosine et la guanine ;
(b) chauffer le mélange réactionnel à une première température prédéterminée pendant un premier temps prédéterminé pour séparer les brins de l'ADN cible les uns des autres ;
(c) refroidir le mélange réactionnel à une deuxième température prédéterminée pendant un deuxième temps prédéterminé dans des conditions permettant les première et deuxième amorces à s'hybrider avec leurs séquences complémentaires sur l'ADN cible et permettant à la polymérase d'étendre les amorces ; et
(d) répéter les étapes (b) et (c) au moins 10 fois, dans lequel une méthylation accrue, par rapport aux tissus d'un deuxième sujet humain qui n'a pas de cancer de la prostate, indique la présence d'un cancer de la prostate ou d'un défaut de champ du cancer de la prostate.

7. Utilisation d'un kit pour le diagnostic du cancer de la prostate ou la détection de défauts sur le terrain du cancer de la prostate, le kit comprenant au moins un ensemble d'amorces, dans lequel l'ensemble d'amorces consiste en une amorce sens et une amorce anti-sens conçues pour amplifier une région cible sélectionnée dans le groupe composé des SEQ ID NO: 18 et 39, et de composants essentiels pour l'amplification de l'ADN.

8. Utilisation du kit selon la revendication 7, dans laquelle l'ensemble d'amorces consiste en une amorce sens sélectionnée dans le groupe constitué des SEQ ID NO: 88 et 91, et une amorce anti-sens sélectionnée dans le groupe constitué des SEQ ID NO: 89 et 92.

9. Utilisation du kit selon la revendication 7, dans laquelle les composants essentiels à l'amplification de l'ADN comprennent la polymérase, les dNTP et un sel de magnésium qui peut libérer Mg²⁺.

10. Utilisation du kit selon la revendication 7, dans laquelle le kit comprend en outre des sondes spécifiques de méthylation et de non-méthylation marquées au fluorophore FAM ou Hex et convient pour un essai en tube fermé pour MS-QPCR.

11. Utilisation du kit selon la revendication 7, dans laquelle le kit comprend en outre des amorces de séquençage et convient pour un test à base de pyroséquençage bisulfite.

12. Utilisation du kit selon la revendication 7, dans laquelle les amorces de séquençage sont choisies dans le groupe constitué des SEQ ID NQ: 90 et 93.

13. Utilisation du kit selon la revendication 7, comprenant en outre des billes de streptavidine sépharose, un mélange d'enzymes, un mélange de substrats et des dinucléotides.

14. Utilisation du kit selon la revendication 7, dans laquelle le kit comprend des ensembles d'amorces sélectionnés dans le groupe constitué de deux ensembles, trois ensembles, quatre ensembles, cinq ensembles, six ensembles, sept ensembles et huit ensembles.
